# EUROPEAN PATENT APPLICATION

(11) **EP 1 598 335 A1**
(43) Date of publication of application: **23.11.2005**
(21) Application number: 04712705.5
(22) Date of filing: 19.02.2004
(51) Int. Cl.: C07C 405/00, A61K 31/5575, A61P 11/02, A61P 11/06, A61P 27/14, A61P 37/08, A61P 43/00

(54) **PROSTAGLANDIN DERIVATIVES**

(30) Priority: 20.02.2003 JP 2003042812; 08.05.2003 JP 2003130632
(71) Applicant: TAISHO PHARMACEUTICAL CO., LTD, Tokyo 170-8633 (JP); Sato, Fumie, Fujisawa-shi, Kanagawa 251-0026 (JP)
(72) Inventor: SATO, Fumie, Fujisawa-shi, Kanagawa 251-0026 (JP); TANAMI, Tohru, c/o Taisho Pharmaceutical Co., Ltd., Tokyo 170-8633 (JP); ONO, Naoya, c/o Taisho Pharmaceutical Co., Ltd., Tokyo 170-8633 (JP); YAGI, Makoto, c/o Taisho Pharmaceutical Co., Ltd., Tokyo 170-8633 (JP); SEKI, Takayuki, c/o Taisho Pharmaceutical Co. Ltd., Tokyo 170-8633 (JP); SATO, Mariko, c/o Taisho Pharmaceutical Co., Ltd., Tokyo 170-8633 (JP)
(74) Representative: HOFFMANN EITLE
(86) International application number: PCT/JP2004/001878
(87) International publication number: WO 2004/074240

(57) **Abstract**

A prostaglandin derivative represented by the formula 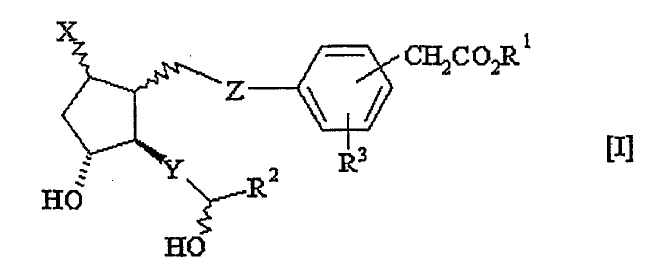 (wherein X is a halogen atom; Y is ethylene group, vinylene group or ethylylene group; Z is a group represented by -(CH₂)ₘ-, -O(CH₂)ₙ- or -S(O)p-(CH₂)ₙ- (m is an integer of 0 to 3; n is an integer of 0 to 2; and p is an integer of 0 to 2); R¹ is a hydrogen atom, a C₁₋₅ alkyl group or a substituted C₁₋₅ alkyl group; R² is a C₃₋₁₀ cycloalkyl group, a C₃₋₁₀ cycloalkyl group substituted by C₁₋₄ alkyl group or C₄₋₁₅ cycloalkylalkyl group; and R³ is a hydrogen atom, a halogen atom or a C₁₋₅ alkyl group or a substituted C₁₋₅ alkyl group), a pharmaceutically acceptable salt thereof or a hydrate thereof which has an excellent antagonism to prostaglandin DP receptor and, therefore, is useful against diseases such as allergic rhinitis, nasal obstruction, asthma, allergic conjunctivitis, systemic mastocytosis and disorder of systemic mast cell activation.

## Description

### TECHNICAL FIELD

The present invention relates to novel prostaglandin derivatives.

### BACKGROUND ART

Since prostaglandin (hereinafter referred to as "PG") exhibits various important physiological actions in a trace amount, natural PG and numerous of its derivatives have been synthesized and their bioactivities have been studied with the intention of an application for medicines, and have been reported in a great number of papers and patents (Japanese laid-open patent publication No. S52-100446 and published Japanese translations of PCT international publication for patent applications No. H2-502009 or the like). PGDs are known to have actions of bronchoconstriction, vascular dilatation or constriction, platelet aggregation inhibition, and so on. PGDs exert their effects by binding to their receptors, DP receptors. DP antagonists bind to the receptors thereof and inhibit the effects of PGD. For example, they are considered to be useful for diseases such as allergic rhinitis, nasal obstruction, asthma, allergic conjunctivitis, systemic mastocytosis and disorder of systemic mast cell activation. Although some DP antagonists are known to date, their actions are hardly adequate. Further, a group of PG derivatives with similar structure substituted with halogen at the 9-position is disclosed (Japanese laid-open patent publication No. H7-233143 and international patent publication No. WO0112596), but they are all PGDs with agonist activity similar to that of PG derivatives and no compound with DP antagonist activity that is a PG derivative substituted with halogen at the 9-position is reported.

The object of the present invention is to provide PG derivatives with excellent DP antagonist activity.

### DISCLOSURE OF THE INVENTION

As a result of the intense investigations, the present inventors have found that novel prostaglandin derivatives represented by the following Formula (I) can achieve the above-mentioned object, and thereby the present invention has been accomplished.

That is, the present invention is directed to a prostaglandin derivative represented by Formula (I): 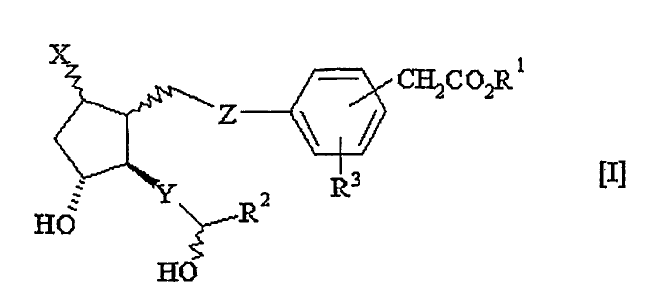 (wherein X is a halogen atom, Y is an ethylene group, vinylene group, or ethynylene group, Z is a group represented by -(CH₂)ₘ-, -O(CH₂)ₙ-, or -S(O)ₚ-(CH₂)ₙ- (wherein m is an integer of 0 to 3, n is an integer of 0 to 2, and p is an integer of 0 to 2), R¹ is a hydrogen atom, a C₁₋₅ alkyl group or a substituted C₁₋₅ alkyl group, R² is a C₃₋₁₀ cycloalkyl group, a C₃₋₁₀ cycloalkyl group substituted with a C₁₋₄ alkyl group, or a C₄₋₁₅ cycloalkylalkyl group, and R³ is a hydrogen atom, a halogen atom, a C₁₋₅ alkyl group, or a substituted C₁₋₅ alkyl group), a pharmaceutically acceptable salt thereof or a hydrate thereof.

In the present invention, the halogen atom is a fluorine atom, a chlorine atom, a bromine atom, or an iodine atom.

The vinylene group is a trans-vinylene group or a cis-vinylene group.

Examples of the C₃₋₁₀ cycloalkyl group are a cyclopropyl group, a cyclobutyl group, a cyclopentyl group, a cyclohexyl group, a cycloheptyl group, a cyclooctyl group, a cyclodecyl group and the like.

Examples of the C₃₋₁₀ cycloalkyl group substituted with a C₁₋₄ alkyl group are a methylcyclopropyl group, a methylcyclohexyl group, an ethylcyclohexyl group, a methylcyclooctyl group, a tert-butylcyclohexyl group and the like.

Examples of the C₄₋₁₀ cycloalkylalkyl group are a cyclopropylmethyl group, a cyclobutylmethyl group, a cyclopentylmethyl group, a cyclopentylethyl group, a cyclohexylmethyl group, a cyclohexylethyl group, a cycloheptylmethyl group, and a 2-cyclohexylprop-2-yl group and the like.

The C₁₋₅ alkyl group is a linear or branched alkyl group, examples of which are a methyl group, an ethyl group, a propyl group, an isopropyl group, a butyl group, an isobutyl group, a tert-butyl group, a pentyl group, an isopentyl group, a 2-methylbutyl group, and the like.

The substituted C₁₋₅ alkyl group is a linear or branched C₁₋₅ alkyl group substituted with one or more kind selected from a group consisting of a halogen atom, a cyano group, and alkoxy groups, examples of which are a 2-chloroethyl group, a cyanomethyl group, a trifluoromethyl group and a 2, 2, 2-trifluoroethyl group.

Examples of the pharmaceutically acceptable salt are salts with alkali metals such as sodium and potassium, salts with alkali earth metals such as calcium and magnesium, salts with ammonia, methylamine, dimethylamine, cyclopentylamine, benzylamine, piperidine, monoethanolamine, diethanolamine, monomethylmonoethanolamine, tromethamine, lysine, or tris(hydroxymethyl)aminomethane, tetraalkylammonium salts and the like.

The compound of the Formula (I) may be prepared, for example, by the methods summarized by the following reaction scheme. 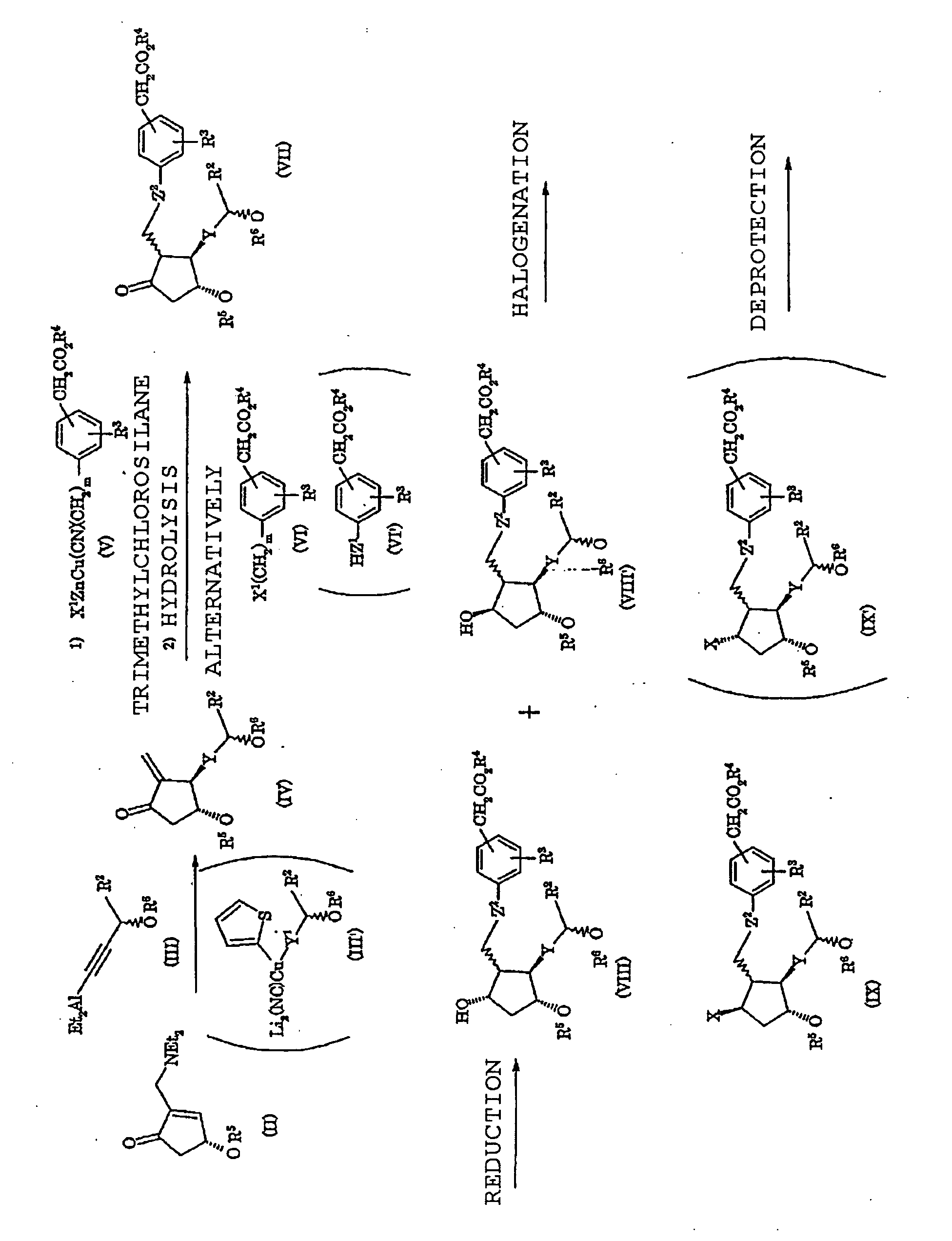 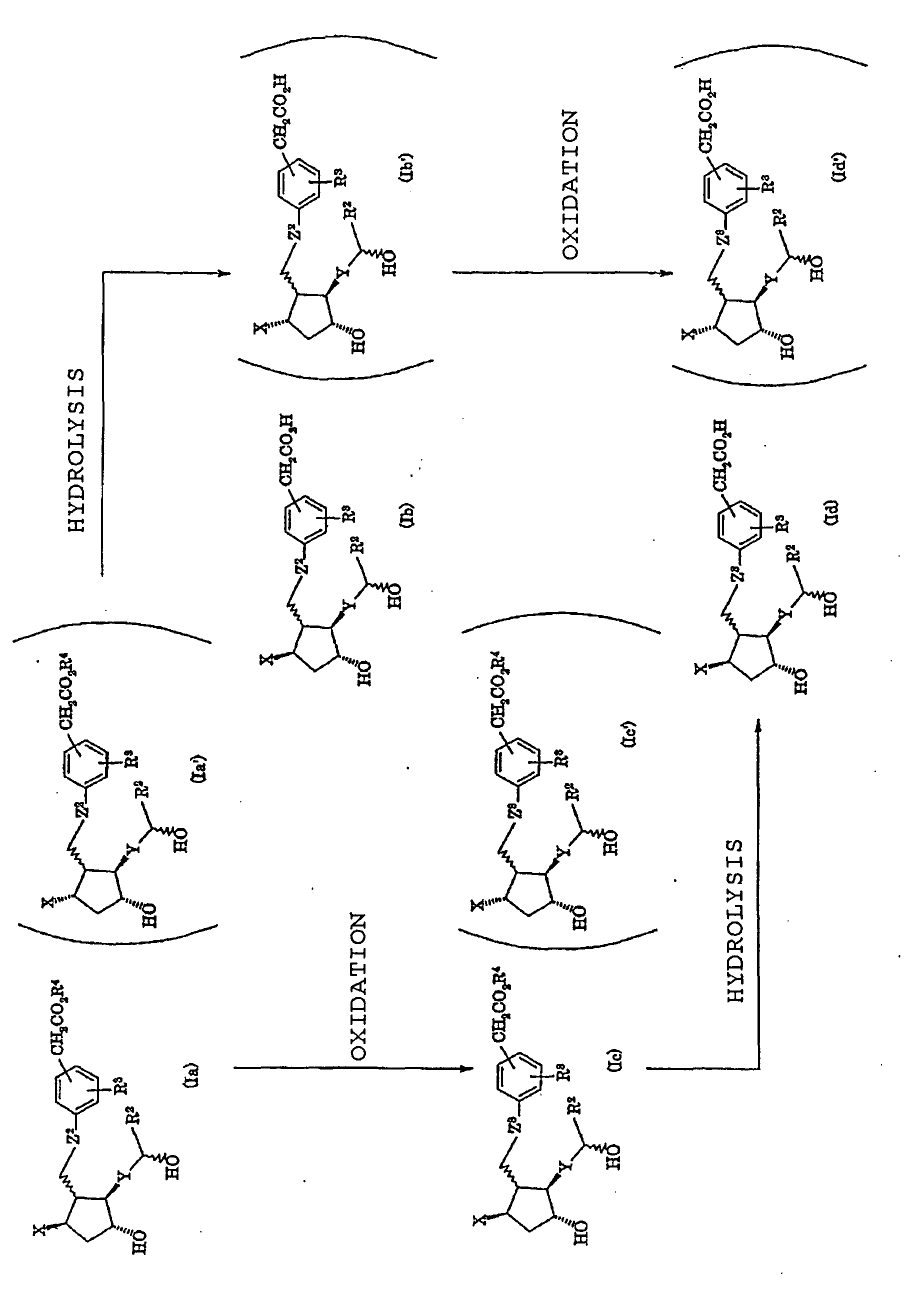 (In the reaction scheme, R⁵ and R⁶ are same or different protective groups for hydroxyl groups, X¹ is a halogen atom other than a fluorine atom, Y¹ is an ethylene group or a vinylene group, Z¹ is an oxygen atom or a sulfur atom, Z² is a group defined by Z wherein p is 0, Z³ is a group defined by Z wherein p is other than 0, R⁴ is a C₁₋₅ alkyl group or a substituted C₁₋₅ alkyl group, and X, R², R³, m, and n have the same meanings of the above mentioned. Herein, the protective groups for hydroxyl groups are those typically used in the field of prostaglandin, such as a tert-butyldimethylsilyl group, a triethylsilyl group, a diphenylmethylsilyl group, a tetrahydropyranyl group, a tetrahydrofuranyl group, a methoxymethyl group, an ethoxyethyl group, and a benzyl group.)

The above-mentioned reaction scheme is illustrated as follows:
(1) At first, a compound of Formula (II), known by the method of Sato et al. (Journal of Organic Chemistry, (J. Org. Chem.) vol . 53, page 5590 (1988)), is reacted with 0.8 to 2.0 equivalents of a compound represented by Formula (III) or (III') in an inert solvent (for instance, benzene, toluene, tetrahydrofuran, diethyl ether, methylene chloride or n-hexane and so forth) at the temperature of -78 to 30 degree C to stereospecifically give a compound of Formula (IV). Herein, the compound wherein Y is an ethylene group or a vinylene group (that is, the compound wherein Y is Y¹) is obtainable by a reaction using a compound of Formula (III') at the temperature of -78 to 0 degree C, and the compound wherein Y is an ethynylene group can be obtained by a reaction using a compound of Formula (III) at the temperature of 0 to 30 degree C.
(2) The compound of Formula (IV) is reacted with 0.5 to 4 equivalents of an organic copper compound represented by Formula (V) and 0.5 to 4.0 equivalents of trimethylchlorosilane in an inert solvent (specifically, benzene, toluene, tetrahydrofuran, diethyl ether, methylene chloride, n-hexane or n-pentane) at the temperature of -78 to 40 degree C, followed by hydrolysis using an inorganic acid (specifically, hydrochloric acid, sulfuric acid or nitric acid or the like), an organic acid (specifically, acetic acid or p-toluenesulfonic acid, and so on) or an amine salt thereof (specifically, pyridinium p-toluenesulfonate) in an organic solvent (specifically, acetone, methanol, ethanol, isopropanol, diethyl ether or a mixture thereof) at the temperature of 0 to 40 degree C to give a compound of Formula (VII).
   Alternatively, the compound of Formula (IV) is reacted with 0.5 to 4 equivalents of a compound represented by Formula (VI), if necessary, 0.05 to 2 equivalents of a radical generator (for example, azobisisobutyronitrile, azobiscyclohexanecarbonitrile, benzoyl peroxide or triethylborane), and further, if necessary, 1 to 5 equivalents of a radical reducing agent (specifically, tributyltin hydride, triphenyltin hydride, dibutyltin hydride or diphenyltin hydride) in an organic solvent (specifically, benzene, toluene, xylene, n-hexane, n-pentane or acetone) at the temperature of -78 to 100 degree C to give a compound of Formula (VII).
   Further alternatively, the compound of Formula (IV) may be reacted with 0.5 to 4 equivalents of a compound represented by Formula (VI'), if necessary, 0.05 to 2 equivalents of a base (for instance, an organic amine such as triethylamine, diisopropylamine, pyridine, and dimethylaniline, or a basic resin such as polyvinylpolypyrrolidone, diisopropylaminomethyl-polystyrene, piperidinomethyl-polystyrene), and further, if necessary, 0.01 to 0.5 equivalents of a complex or a complex salt of divalent palladium (specifically, dichlorobis(acetonitrile)palladium (II), dichlorobis(benzonitrile)palladium (II) or palladium chloride) in an organic solvent (specifically, benzene, toluene, xylene, n-hexane, n-pentane or acetone) at the temperature of -78 to 100 degree C to give a compound of Formula (VII).
   Herein, the organocopper compound of Formula (V) can be prepared from the compound represented by Formula (V') 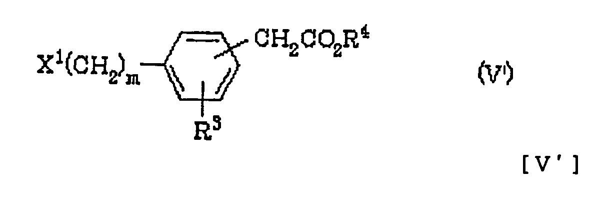 (Wherein X¹, R³, R⁴, and m have the same meanings of the above mentioned) based on the known method [P. Knochel et al., Journal of Organic Chemistry (J. Org. Chem.), vol. 53, page 2390 (1988), and Chemical Reviews (Chem. Rev.), vol. 93, page 2117 (1993)]. That is, the compound of Formula (V') is reacted with, for example, 0.8 to 5 equivalents of zinc activated with 1,2-dibromoethane, trimethylchlorosilane or iodine in an inert solvent (specifically, tetrahydrofuran, diethyl ether, n-hexane, n-pentane, dioxane or dimethoxyethane) to lead an organozinc compound represented by Formula (V") 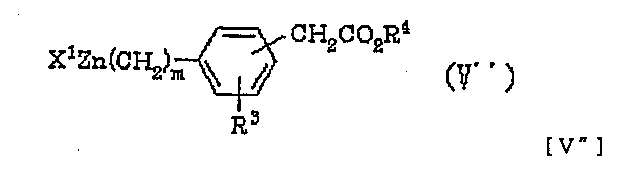 (Wherein X¹, R³, R⁴, and m have the same meanings of the above mentioned). The reaction temperature here is typically 0 to 150 degree C. The organozinc compound obtainable is reacted in the above inorganic solvent containing copper cyanide (1 to 2.5equivalent) and lithium chloride (2 to 5 equivalents) to give an organocopper compound of Formula (V).
(3) The compound of Formula (VII) is reacted with 0.5 to 5 equivalents of a reducing agent such as potassium borohydride, sodium borohydride, lithium tricyanoborohydride, lithium tri-sec-butylborohydride or diisobutylaluminum hydride-BHT (2, 6-di-tert-butyl-p-cresol) in an organic solvent (for example, tetrahydrofuran, diethyl ether, ethyl alcohol or methyl alcohol) at the temperature of -78 to 40 degree C to give compounds of Formula (VIII) and Formula (VIII'). These compounds of Formula (VIII) and Formula (VIII') may be purified by typically used separation methods such as column chromatography.
(4) After mesylation or tosylation of the compound of Formula (VIII) (or Formula (VIII')) with, for example, 1 to 6 equivalents of methanesulfonyl chloride or p-toluenesulfonyl chloride in an appropriate solvent such as pyridine or toluene, if necessary, in the presence of 0.8 to 6 equivalents of a base such as triethylamine or 4-dimethylaminopyridine, chlorination with 1 to 16 equivalents of tetra-n-butylammonium chloride is accomplished to give a compound of Formula (IX) (or Formula (IX')) (X is a chlorine atom). Herein, bromination and fluorination may also be accomplished by usual methods. Bromination, for example, may be accomplished by using 1 to 10 equivalents of carbon tetrabromide in the presence of 1 to 10 equivalents of triphenylphosphine and 1 to 10 equivalents of pyridine in acetonitrile. Fluorination, for example, may be accomplished by reaction with 5 to 20 equivalents of diethylaminosulfur trifluoride (DAST) in methylene chloride.
(5) The hydroxyl group of the compound of Formula (IX) (or Formula (IX')) is deprotected by using tetrabutylammonium fluoride, hydrofluoric acid, pyridinium poly (hydrogen fluoride) or hydrochloric acid and the like in methanol, ethanol, acetonitrile, solvent mixtures thereof or solvent mixtures thereof and of water under typically used conditions to give a PG derivative of formula (Ia) or (Formula (Ia')) related to the present invention.
(6) The compound of Formula (Ia) (or Formula (Ia')) is hydrolyzed using 1 to 6 equivalents of a base in a solvent typically used for hydrolysis to give a PG derivative of Formula (Ib) (or Formula (Ib')) (in Formula (I), R¹ is a hydrogen atom) related to the present invention. As the base, lithium hydroxide and potassium carbonate or the like are exemplified, and as the solvent, acetonitrile, acetone, methanol, ethanol, water or solvent mixtures thereof and so on are exemplified.
   Alternatively, the compound of Formula (Ia) (or (Ia')) is hydrolyzed by a reaction with an enzyme in a buffer solution such as phosphate buffer or tris-hydrochloride buffer, if necessary, by using an organic solvent (specifically, a water-miscible solvent such as acetone, methanol or ethanol) to give a PG derivative of Formula (Ib) (or (Ib')), wherein R¹ is a hydrogen atom in Formula (I), related to the present invention.
   The enzyme may be enzymes produced by microorganisms (specifically, enzymes produced by microorganisms belonging to Candida sp. or Pseudomonas sp.), enzymes prepared from animal organs (specifically, enzymes prepared from pig liver or pig pancreas), or the like, and as commercially available enzymes, lipase VII (produced by Sigma Co.; derived from microorganism of Candida sp.), lipase AY (produced by Amano Pharmaceutical Co.; derived from microorganism of Candida sp.), lipase PS (produced by Amano Pharmaceutical Co.; derived from microorganism of Pseudomonas sp.), lipase MF (produced by Amano Pharmaceutical Co.; derived from microorganism of Pseudomonas sp.), PLE (produced by Sigma Co.; prepared from pig liver), lipase II (produced by Sigma Co.; prepared from pig pancreas) and lipoprotein lipase (produced by Tokyo Kasei Kogyo Co.; prepared from pig pancreas), or the like are exemplified.
   The amount of the enzyme to be used, while depending on the potency of the enzyme and the amount of the substrate [the compound of Formula (Ia)], is usually 0.1 to 20 parts by weight based on the substrate, and the reaction temperature is from 25 to 50 degree C, preferably from 30 to 40 degree C.
(7) The compound of Formula (Ia) (or (Ia')) is reacted using an oxidant such as sodium metaperiodate, hydrogen peroxide solution, peracetic acid, m-chloroperbenzoic acid or tert-butyl hydroxyperoxide in diethyl ether, methanol, ethanol, methylene chloride, water or mixtures thereof at the temperature of -20 to 50 degree C to give a PG derivative of Formula (Ic) (or (Ic')).
(8) The compound of Formula (Ic) (or Formula (Ic')) is hydrolyzed in the similar manner as described in the above (6) to give a PG derivative of Formula (Id) (or Formula (Id')) of the present invention. In addition, oxidation using the compound of Formula (Ib) (or Formula (Ib')) in the similar manner as described in (7) above gives a PG derivative of Formula (Id) or (Id') of the present invention.

The compounds of the present invention may be administered systemically or topically; orally or parenterally such as intravenously and transnasally. They can be orally administered, for example, in the form of tablets, powders, granules, dusting powders, capsules, solutions, emulsions or suspensions, each of which can be prepared according to usual methods. As the dosage form for intravenous administration, aqueous or non-aqueous solutions, emulsions, suspensions or solid preparations to be dissolved in a solvent for injection immediately before use can be used. For transnasal administration, generally solutions and powders (hard capsules) containing drugs are transnasally spray-(atomization-)administered quantitatively using an exclusive nasal sprayer or atomizer. Furthermore, the compounds according to the present invention may be prepared into the form of inclusion compounds with α-, β- or γ-cyclodextrin, or methylated cyclodextrin. In addition, the aqueous or non-aqueous solutions, emulsions, suspensions, or the like thereof, may be administered by injection. The dose is varied by the age, body weight, and so forth, but it is generally from 1 ng to 1000 mg/day per adult, which can be administered in a single dose or divided doses per day.

Representative compounds of Formula (I) according to the present invention are shown as follows.

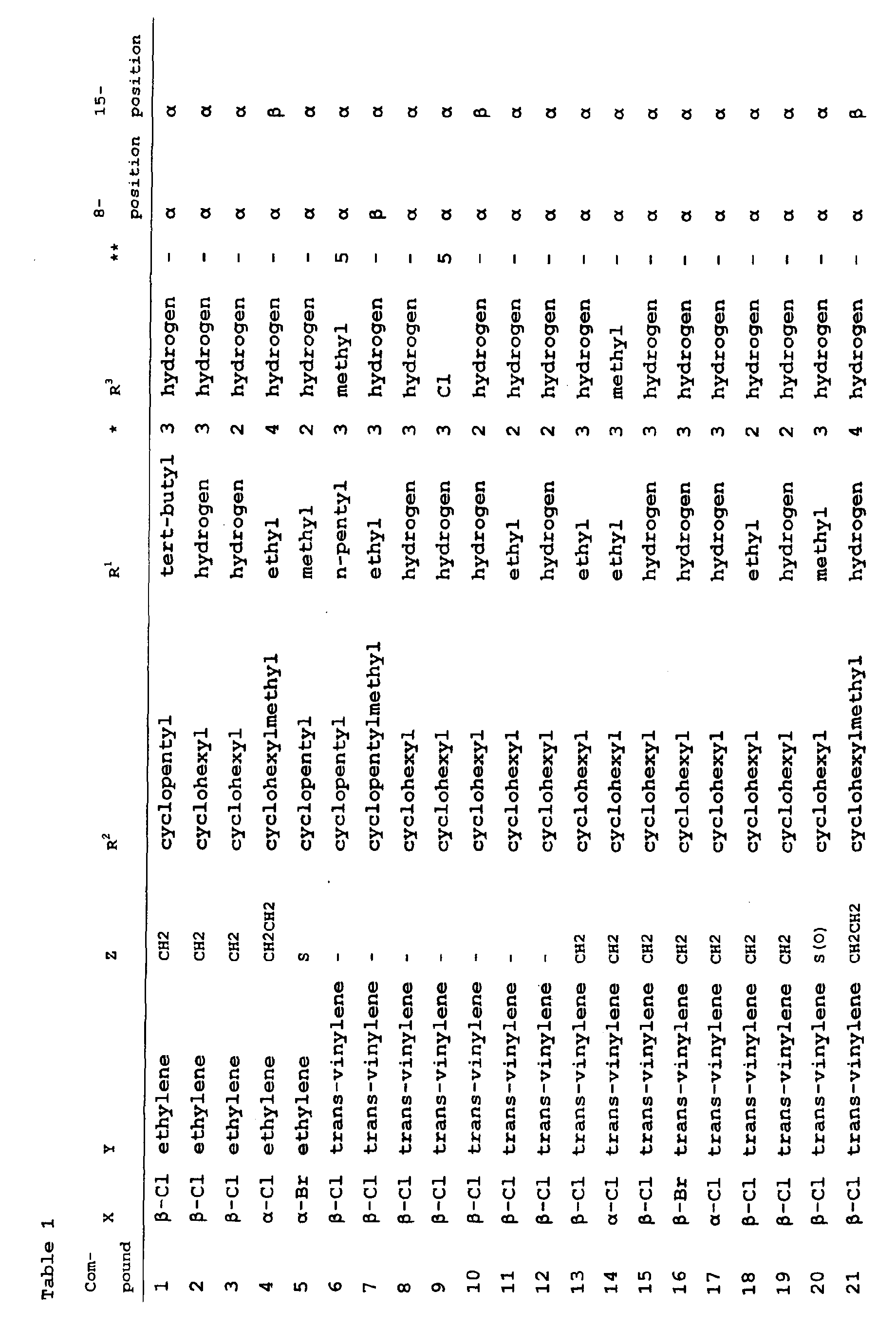

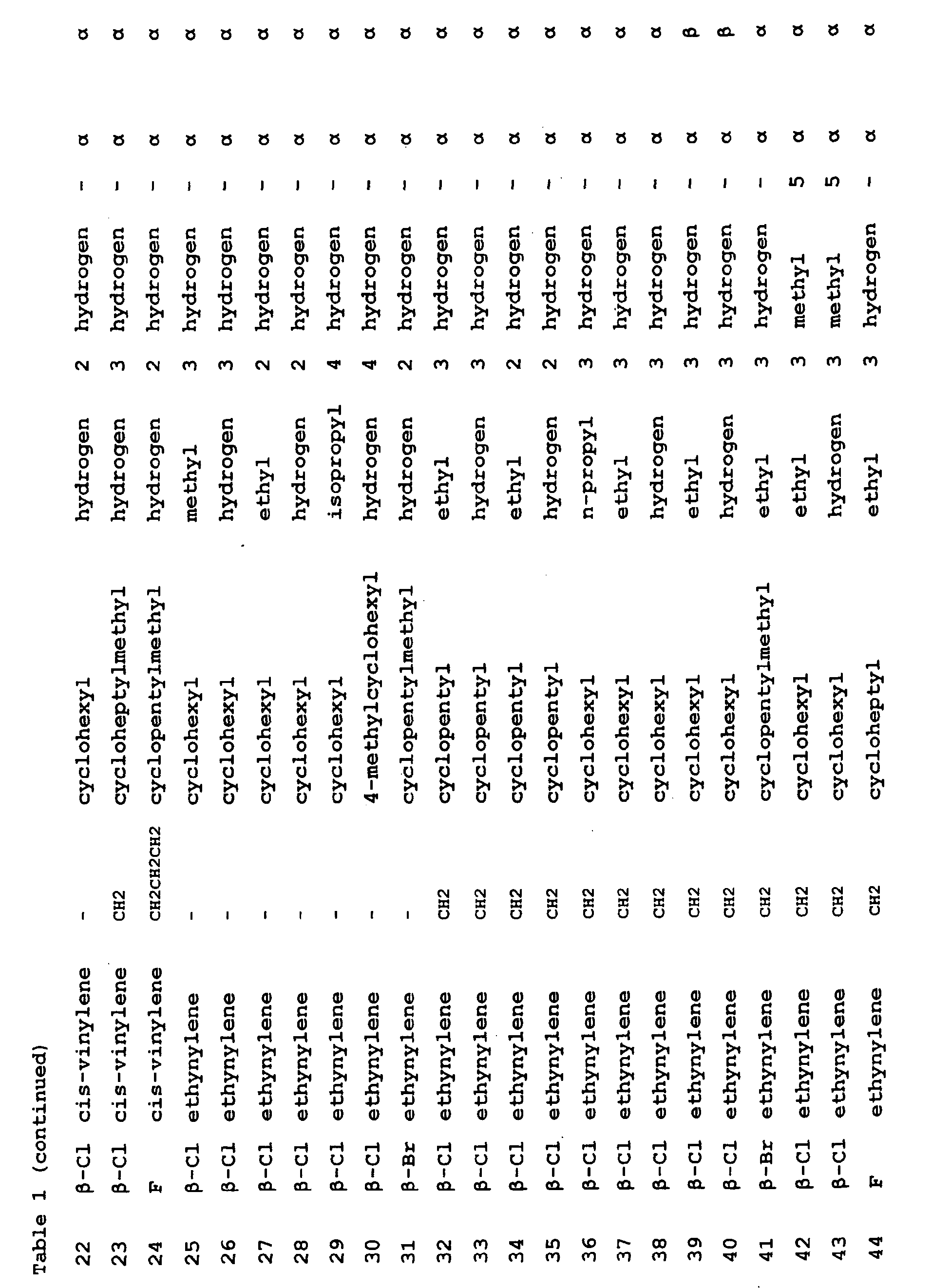

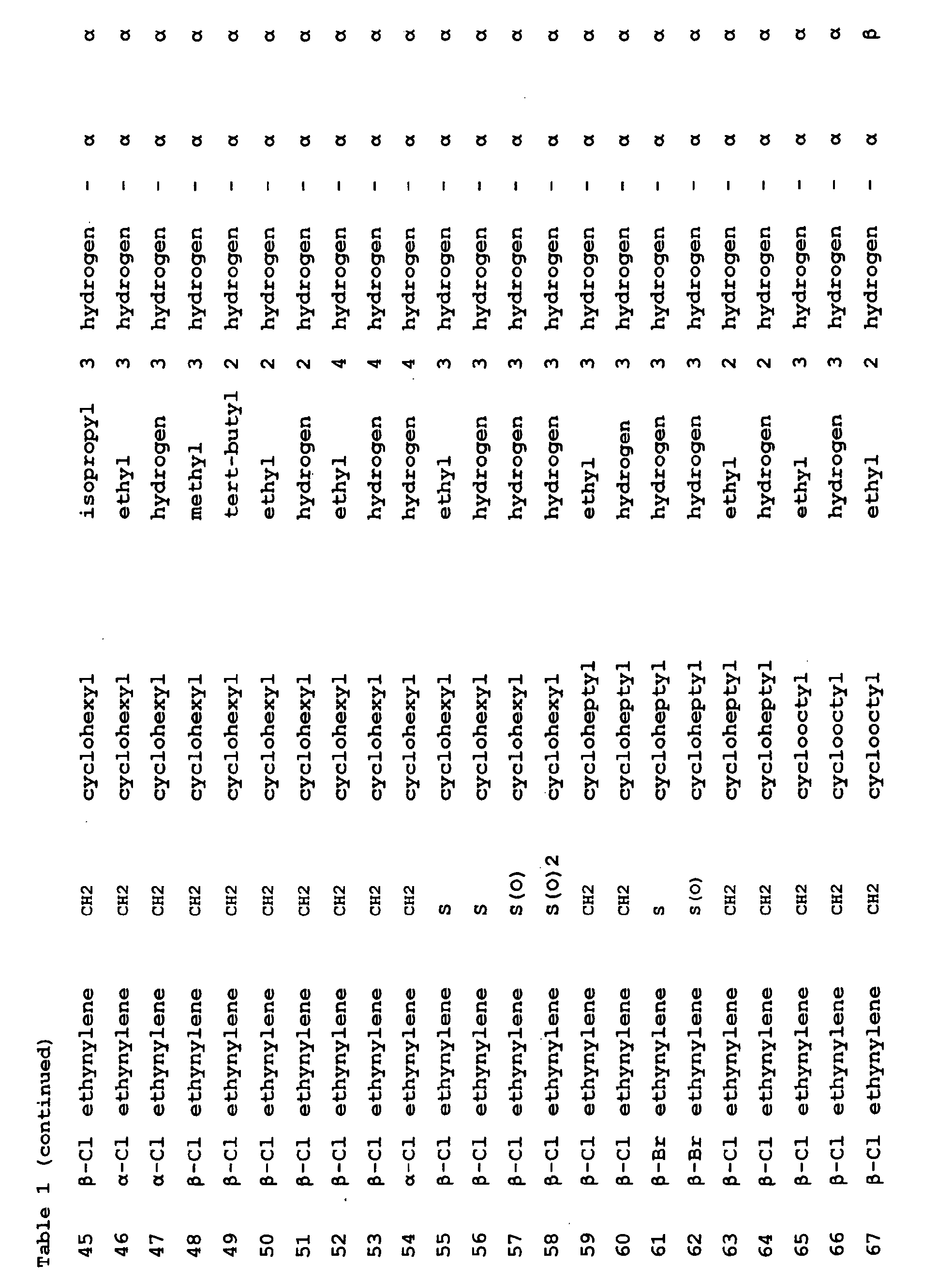

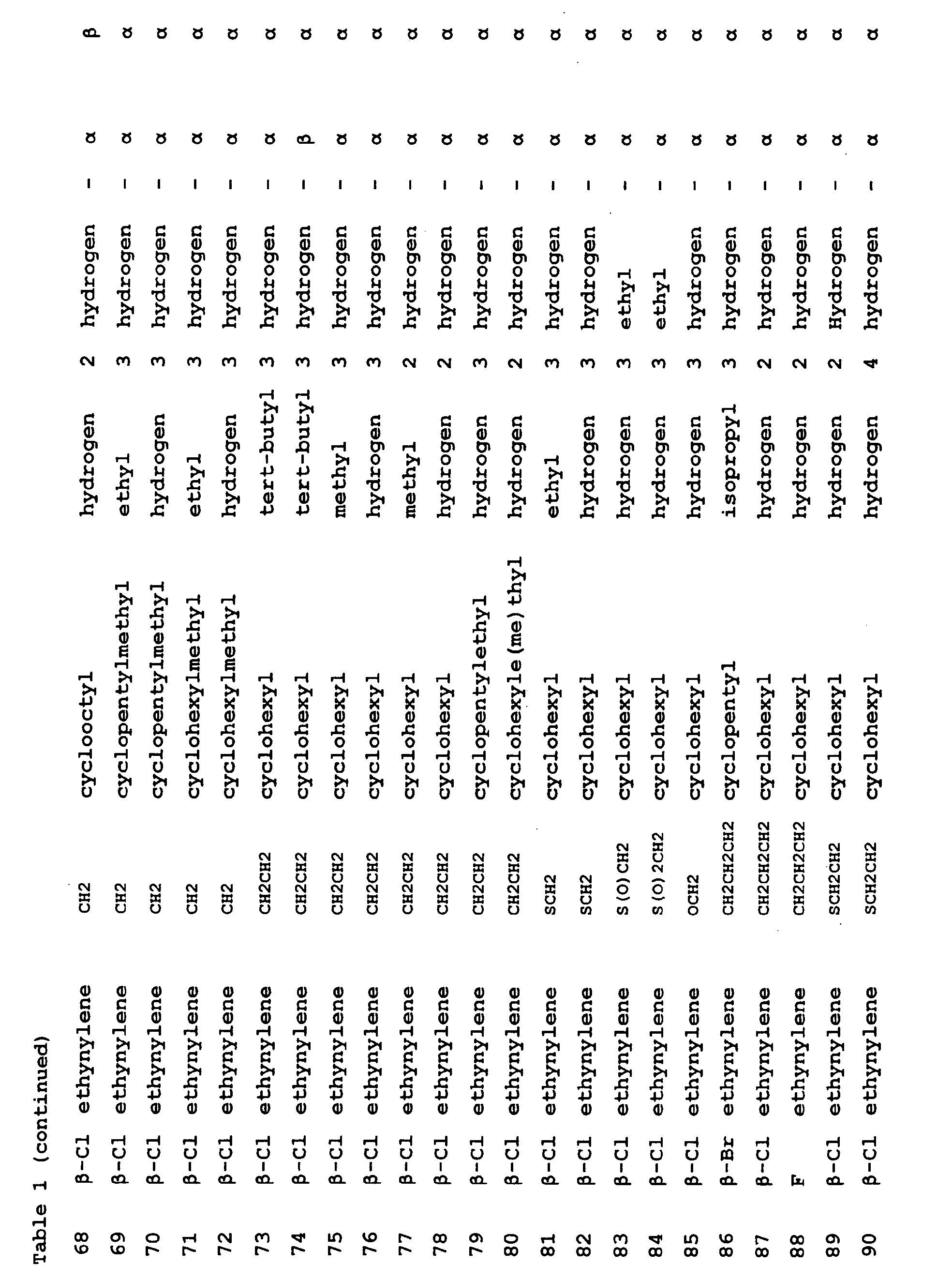

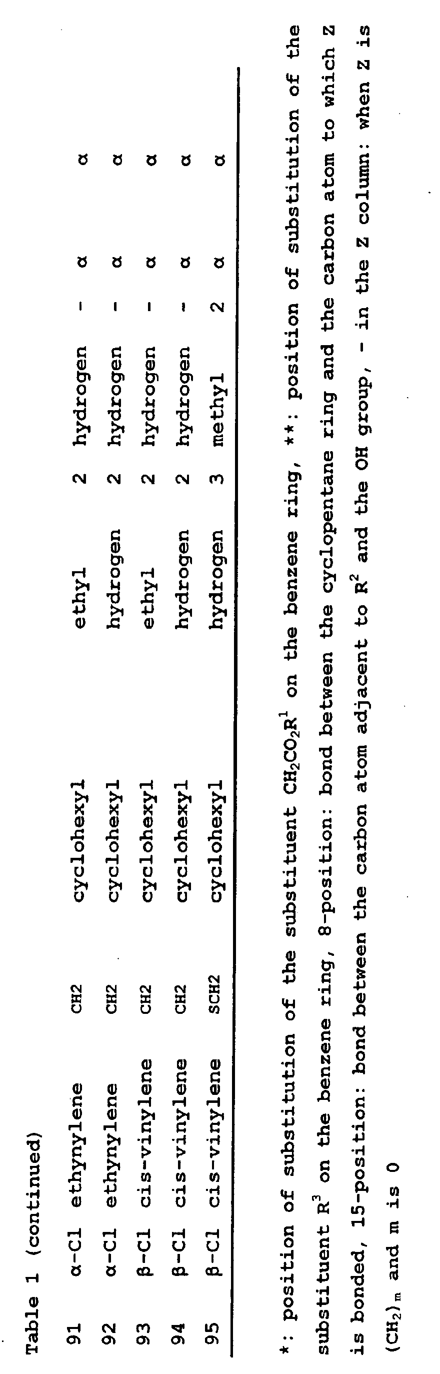

### BEST MODE FOR CARRYING OUT THE INVENTION

The present invention is illustrated in more details by the following examples and experiments.

### Example 1

### 9-Deoxy-9β-chloro-3, 4, 5, 16, 17, 18, 19, 20-octanol-2, 6-inter-m-phenylene-15-cyclohexyl-13, 14-didehydro-PGF₁α ethyl ester (Compound 37)

(1) (3S)-3-(tert-butyldimethylsil,oxy)-3-cyclohexylp rop-1-yne (16.4 g) was dissolved in 200 ml of toluene, and n-butyl lithium (2.46 M, hexane solution, 24.4 ml) was added at 0 degree C, followed by stirring at the same temperature for 30 minutes. Diethylaluminum chloride (0.93 M, hexane solution, 75.3 ml) was added to the solution at 0 degree C, followed by stirring for 30 minutes up to room temperature.
   (4R)-2-(N,N-diethylamino)methyl-4-(tert-butyldimethy lsiloxy)cyclopent-2-en-1-one (0.25 M, toluene solution, 200 ml) was added to the solution at room temperature followed by stirring for 15 minutes. The reaction solution was poured into a mixture of hexane (485 ml) - a saturated aqueous ammonium chloride solution (485 ml) - aqueous hydrochloric acid solution (3M, 140 ml) under stirring, and the organic layer was separated, which was washed with a saturated aqueous sodium hydrogen carbonate solution (100 ml). The resulting organic layer was dried over anhydrous magnesium sulfate, filtered and concentrated, and the residue was purified by a silica column chromatography (developing solvent; hexane:ether = 10:1) to give (3R, 4R)-2-methylene-3-[(3S)-3-(tert-butyldimethylsiloxy) -3-cyclohexylprop-1-ynyl]-4-(tert-butyldimethylsilox y)cyclopentane-1-one (19.8 g).
   ¹H-NMR(CDCl₃ 200MHz) δ ppm; 0.08 (s, 3H), 0.10 (s, 6H), 0.13 (s, 3H), 0.83-1.92 (m, 11H), 0.90 (s, 18H), 2.33 (dd, J=17.9, 7.4Hz, 1H), 2.72 (dd, J=17.9, 6.5Hz, 1H), 3.49-3.58 (m, 1H), 4.13 (dd, J=6.1, 1.6Hz, 1H), 4.22-4.34 (m, 1H), 5.56 (dd, J=2.7, 0.7Hz, 1H), 6.14, (dd, J=3.1, 0.7Hz, 1H)
   IR(neat): 2929, 2856, 2234, 1737, 1645, 1473, 1463, 1451, 1381, 1362, 1245, 1104, 1006, 899, 838, 778, 669 cm⁻¹
(2) Copper(I) cyanide-2 lithium chloride (1.0 M, tetrahydrofuran solution, 39.4 ml) was added to 3-carboethoxymethylbenzylzinc(II) bromide (0.80 M, tetrahydrofuran solution, 38.0 ml) at -70 degree C followed by stirring for 20 minutes at the same temperature. To the solution was added the compound obtained in the above (1) (0.25 M, diethyl ether solution, 78.8 ml) and chlorotrimethylsilane (2.6 ml, 20.2 mmol) at -70 degree C, and the temperature was raised to 0 degree C with stirring over about 1 hour. To the reaction solution was added 300 ml of a saturated aqueous ammonium chloride solution, followed by extraction with hexane. The organic layer was washed with a saturated aqueous sodium chloride solution, dried and concentrated, and the resulting residue was dissolved in diethyl ether (19.7 ml)-isopropyl alcohol (78.8 ml), followed by addition of pyridinium p-toluenesulfonate (99 mg) and stirring at room temperature for 12 hours . The reaction solution, after addition of hexane (200 ml), was washed with a saturated aqueous sodium hydrogen carbonate solution and a saturated aqueous sodium chloride solution, dried, filtered and concentrated, and the resulting residue was purified by a silica gel column chromatography (developing solvent; hexane:ethyl acetate=15:1) to give 3, 4, 5, 16, 17, 18, 19, 20-octanol-2, 6-inter-m-phenylene-15-cyclohexyl-13, 14-didehydro-PGE₁ ethyl ester 11,
   15-bis(tert-butyldimethylsilyl ether) (9.75 g).
   ¹H-NMR (CDCl₃, 300MHz) δ ppm; 0.08 (s, 3H), 0.09 (s, 3H), 0.10 (s, 3H), 0.13 (s, 3H), 0.80-2.30 (m, 14H), 0.90 (s, 18H), 1.25 (t, J=7.2Hz, 3H), 2.20 (dd, J=18.3, 6.8Hz, 1H), 2.63-2.85 (m, 4H), 3.57 (s, 2H) 4.09 (dd, J=6.4, 1.6Hz, 1H), 4.14 (q, J=7.2Hz, 2H), 4.25-4.36 (m, 1H), 7.02-7.29 (m, 4H),
   IR (neat) : 3400, 2929, 2856, 2237, 1746, 1609, 1472, 1463, 1450, 1385, 1369, 1337, 1252, 1100, 1070, 1034, 1006, 939, 898, 883, 838, 778, 700, 670 cm⁻¹
(3) Diisobutylaluminum hydride (0.93 M hexane solution, 35.2 ml) was added to a toluene (49.5 ml) solution of BHT (2,6-di-tert-butyl-p-cresol) (7.78 g) at -78 degree C followed by stirring for 1 hour at -10 degree C. To the reaction solution was added by drop a toluene (25 ml) solution of the compound (9.75 g) obtained in the above (2) at -78 degree C followed by stirring for 1 hour at -25 degree C. To the reaction solution was added a saturated aqueous ammonium chloride solution, and the mixture was made weakly acidic with 1 M aqueous hydrochloric acid and extracted with ethyl acetate. The organic layer was washed with 1 M aqueous hydrochloric acid and a saturated aqueous sodium chloride solution, dried over anhydrous magnesium sulfate and filtered. The filtrate was concentrated under reduced pressure, and the resulting residue was purified by a silica gel column chromatography (developing solvent; n-hexane: ethyl acetate = 20: 1 to 6: 1) to give 3, 4, 5, 16, 17, 18, 19, 20-octanol-2, 6-inter-m-phenylene-15-cyclohexyl-13, 14-didehydro-PGF₁α ethyl ester 11, 15-bis(tert-butyldimethylsilyl ether) (7.67 g)
   ¹H-NMR (CDCl₃, 300MHz) δ ppm; 0.07 (s, 3H), 0.09 (s, 6H), 0.11 (s, 3H), 0.76-2.07 (m, 16H), 0.89 (s, 9H), 0.90 (s, 9H), 1.25 (t, J=7.2Hz, 3H), 2.40-2.85 (m, 4H), 3.58 (s, 2H), 4.07 (dd, J=6.3, 1.8Hz, 1H), 4.04-4.20 (m, 1H), 4.15 (q, J=7.2Hz, 2H), 4.25-4.31 (m, 1H), 7.07-7.17 (m, 3H), 7.18-7.24 (m, 1H)
   IR (neat) : 3468, 2929, 2856, 2232, 1739, 1609, 1472, 1450, 1389, 1368, 1337, 1252, 1102, 1065, 1006, 898, 837, 777, 701, 668 cm⁻¹
   and 3, 4, 5, 16, 17, 18, 19, 20-octanol-2, 6-inter-m-phenylene-15-cyclohexyl-13, 14-didehydro-PGF₁β ethyl ester 11, 15-bis(tert-butyldimethylsilyl ether) (0.87 g).
   ¹H-NMR(CDCl₃, 300MHz) δ ppm; 0.07 (s, 3H), 0.08 (s, 3H), 0.09 (s, 3H), 0.11 (s, 3H), 0.82-1.98.(m, 17H), 0.88 (s, 9H), 0.90 (s. 9H), 1.25 (t, J=7.2Hz, 3H), 2.25-2.37 (m, 1H), 2.67-2.85 (m, 2H), 3.58 - (s, 2H), 3.93-4.04 (m, 1H), 4.09 (dd, J=6.1, 1.6Hz, 1H), 4.15 (q, J=7.2Hz, 2H), 4.19-4.29 (m, 1H), 7.07-7.16 (m, 3H), 7.20-7.25 (m, 1H)
   IR(heat):3436, 2929, 2855, 2233, 1739, 1609, 1472, 1463, 1450, 1370, 1337, 1252, 1102, 1068, 1006, 939, 898, 837, 777, 700 cm⁻¹
(4) To a pyridine (117 ml) solution of 3, 4, 5, 16, 17, 18, 19, 20-octanol-2, 6-inter-m-phenylene-15-cyclohexyl-13, 14-didehydro-PGF₁α ethyl ester 11, 15-bis(tert-butyldimethylsilyl ether) (7.67g) obtained in (3) was added methanesulfonyl chloride (1.81 ml) at 0 degree C, followed by stirring for 2 hours after elevating temperature to room temperature. After adding toluene (117 ml) thereto, n-Bu₄NCl (19.5 g) was added followed by stirring for 16 hours at 45 degree C. After addition of a saturated aqueous sodium chloride solution (300 ml), the mixture was extracted with hexane (300 ml). The resulting organic layer was washed with 1 M hydrochloric acid and a saturated aqueous sodium chloride solution, dried over anhydrous magnesium sulfate, and filtered. The filtrate was concentrated under reduced pressure and the resulting crude product was purified by a silica gel column chromatography (developing solvent; hexane: ethyl acetate=50: 1) to give 9-deoxy-98-chloro-3, 4, 5, 16, 17, 18, 19, 20-octanol-2, 6-inter-m-phenylene-15-cyclohexyl-13, 14-didehydro-PGF₁α ethyl ester 11, 15-bis(tert-butyldimethylsilyl ether) (6.89 g).
   ¹H-NMR (CDCl₃, 300MHz) δ ppm; 0.08 (s, 6H), 0.10 (s, 3H) 0.11 (s, 3H), 0.82-2.22 (m, 16H, 0.88 (s, 9H), 0.90 (s, 9H), 1.25 (t, J=7.2Hz, 3H), 2.33-2.41 (m, 1H), (m, 16H), 0.88 (s, 9H) 0.90 (s, 9H), 1.25 (t, J=7.2Hz, 3H), 2.33-2.41 (m, 1H), 2.67-2.85 (m, 2H), 3.59 (s, 2H), 3.96-4.06 (m, 1H), 4.09 (dd, J=6.3, 1.5Hz, 1H), 4.15 (q, J=7.2Hz, 2H), 4.23-4.31 (m, 1H), 7.06-7.28 (m, 4H)
   IR(neat) : 2929, 2856, 2235, 1740, 1609, 1472, 1463, 1450, 1389, 1367, 1337, 1252, 1150, 1100, 1034, 1006, 939, 898, 837, 777, 700, 669 cm⁻¹
(5) Concentrated hydrochloric acid (0.50 ml) was added to an ethanol (102 ml) solution of the compound (6.89 g) obtained in (4) followed by stirring at room temperature for 2 days. This was poured into a saturated aqueous sodium hydrogen carbonate solution (100 ml) and extracted twice with ethyl acetate (200 ml). The resulting organic layer was washed with a saturated aqueous sodium chloride solution, dried over anhydrous magnesium sulfate and filtered. The filtrate was concentrated under reduced pressure, and the resulting crude product was purified by a silica gel column chromatography (developing solvent; hexane: ethyl acetate=1: 1 to 1: 2) to give the title compound (4.39 g).
   ¹H-NMR(CDCl₃, 300MHz) δ ppw; 0.96-1.34 (m, 5H), 1.26 (t, J=7.2Hz, 3H), 1.45-2.41 (m, 13H), 2.37 (ddd, J=9.8, 6.4, 1.7Hz, 1H), 2.70-2.89 (m, 2H), 3.59 (s, 2H), 3.96-4.21 (m, 2H), 4.15 (q, J=7.2Hz, 2H), 4.30-4.42 (m, 1H), 7.06-7.29 (m, 4H)
   IR (neat): 3357, 2932, 2856, 2237, 1735, 1611, 1451, 1417, 1369, 1336, 1306, 1248, 1152, 1115, 1096, 1034, 1017, 892, 828, 787, 720, 696, 584, 508, 447 cm⁻¹

### Example 2

### 9-Deoxy-9β-chloro-3, 4, 5, 16, 17, 18, 19, 20-octanol-2, 6-inter-m-phenylene-15-cyclohexyl-13, 14-didehydro-PGF₁α (Compound 38)

To a methanol (52 ml) solution of the compound (2.16 g) obtained in Example 1 was added water (5.2 ml) and lithium hydroxide monohydrate (1.08 g) followed by stirring at room temperature for 3 hours. The reaction solution was made weakly acidic using hydrochloric acid (2M), salted out by addition of sodium hydrogen sulfate, extracted with diethyl ether, dried over anhydrous magnesium sulfate and filtered. The crude product obtained by concentration of the filtrate under reduced pressure was purified by a silica gel column chromatography (developing solvent; ethyl acetate) to give the title compound (2.08 g).
¹H-NMR(CDCl₃, 300MHz) δ ppm ; 0.92-2.38 (m, 16H), 2.33 (ddd, J=9.8, 6.7, 1.9Hz, 1H), 2.70-2.89 (m, 2H), 3.61 (s, 2H), 3.80-5.10 (m, 5H), 4.16 (dd, J=6.1, 1.9Hz, 1H), 7.05-7.29 (m, 4H)
IR(neat) : 3390, 2929, 2854, 2664, 2237, 1715, 1609, 1590, 1489, 1450, 1412, 1262, 1154, 1084, 1008, 894, 758, 704, 668, 615 cm⁻¹

### Example 3

### 9-Deoxy-9β-chloro-3, 4, 5, 17, 18, 19, 20-heptanol-2, 6-inter-m-phenylene-16-cyclopentyl-13, 14-didehydro-PGF₁α ethyl ester (Compound 69)

(1) Following the substantially same manner as in Example 1(1) using
   (3S)-3-(tert-butyldimethylsiloxy)-4-cyclopentylbut-1 -yne in place of
   (3S)-3-(tert-butyldimethylsiloxy)-3-cyclohexylprop-1 -yne in Example 1(1), thereby (3R, 4R)-2-methylene-3-[(3S)-3-(tert-butyldimethylsiloxy) -4-cyclopentylbut-1-ynyl]-4-(tert-butyldimethylsilox y)cyclopentane-1-one was obtained.
   ¹H-NMR (CDCl₃, 300MHz) δppm; 0.07-0.17 (m, 12H), 0.89 (s, 18H), 1.03-2.02.(m, 11H), 2.33(dd, J=17.9, 7.6Hz, 1H), 2.71 (dd, J=17.9, 6.4Hz, 1H), 3.41-3.58 (m, 1H), 4.22-4.31 (m, 1H), 4.39 (t, J=6.7Hz, 1H), 5.55 (d, J=2.4Hz, 1H) 6.14(d, J=3.0Hz, 1H)
   IR(neat): 2930, 2850, 1735, 1638, 1460, 1360, 1245, 1220, 1100, 1000, 935, 825, 770 cm⁻¹
(2) Following the substantially same manner as in Example 1(2) using the compound obtained in (1) above, thereby 3, 4, 5, 17, 18, 19, 20-heptanol-2, 6-inter-m-phenylene-16-cyclopentyl-13, 14-didehydro-PGE₁ ethyl ester 11, 15-bis(tert-butyldimethylsilyl ether) was obtained.
   ¹H-NMR(CDCl₃, 300MHz) δppm; 0.10 (s, 6H), 0.11 (s, 3H), 0.14, (s, 3H), 0.81-2.35 (m, 14H), 0.90, (s, 18H), 2.20 (dd, J=18.3, 7.0Hz, 1H), 2.61-2.85 (m, 4H), 3.57 (s, 2H), 4.15 (q, J=7.2Hz, 2H), 4.25-4.41 (m, 2H), 7.06-7.15 (m, 3H), 7.18-7.27 (m, 1H)
   IR(neat): 2952, 2930, 2858, 2236, 1746, 1610, 1472, 1385, 1368, 1336, 1252, 1150, 1098, 1034, 1006, 939, 838, 778, 700, 670 cm⁻¹
(3) Following the substantially same manner as in Example 1(3) using the compound obtained in (2) above, thereby 3, 4, 5, 17, 18, 19, 20-heptanol-2, 6-inter-m-phenylene-16-cyclopentyl-13, 14-didehydro-PGF₁α ethyl ester 11, 15-bis(tert-butyldimethylsilyl ether) was obtained.
   ¹H-NMR (CDCl₃, 200MHz) δ ppm; 0.09 (s, 6H), 0.10 (s, 6H), 0.76-2.08 (m, 16H), 0.88 (s, 9H), 0.90 (s, 9H), 1.25 (t, J=7.1Hz, 3H), 2.48-2.90 (m, 4H), 3.58 (s, 2H), 4,06-4.41 (m, 2H), 4.15 (q, J=7.1Hz, 2H), 4.35 (dt, J=1.8, 6.9Hz, 1H), 7.04.-7.30-(m, 4H)
   IR(neat) : 3468, 2952, 2930, 2857, 2231, 1739, 1609, 1472, 1388, 1367, 1336, 1253, 1132, 1101, 1073, 1034, 1005, 959, 837, 777, 701, 668 cm⁻¹
(4) Following the substantially same manner as in Example 1(4) using the compound obtained in (3) above, thereby 9-deoxy-9β-chloro-3, 4, 5, 17, 18, 19, 20-heptanol-2, 6-inter-m-phenylene-16-cyclopentyl-13, 14-didehydro-PGF₁α ethyl ester 11, 15-bis(tert-butyldimethylsilyl ether) was obtained.
   ¹H-NMR(CDCl₃, 300MHz) δppm; 0.08 (s, 3H), 0.10 (s, 6H), 0.12, (s, 3H), 0.88 (s, 9H), 0.90 (s, 9H), 1.02-1.19 (m, 2H), 1.26 (t, J=7.2Hz, 3H), 1.43- 2.05 (m, 12H), 2.09-2.21 (m, 2H), 2.32-2.40 (m, 1H), 2.66-286 (m, 2H), 3.59 (s, 2H), 3.96-4.06 (m, 1H), 4.15 (q, J=7.2Hz, 2H), 4.23-4.31 (m, 1H), 4.36 (dt, J=1.9, 6.4Hz, 1H), 7.08-7.15 (m, 3H), 7.20-7.29 (m, 1H)
   IR(neat): 3400, 2952, 2930, 2858, 2232, 1740, 1609, 1472, 1385, 1366, 1253, 1150, 1078, 1035, 1006, 939, 837, 777, 700, 669 cm⁻¹
(5) Following the substantially same manner as in Example 1(5) using the compound obtained in (4) above, thereby the title compound was obtained.
   ¹H-NMR(CDCl₃, 300MHz) δ ppm, 1.04-130 (m, 2H), 1.26 (t, J=7.2Hz, 3H) 1.41-2.40 (m, 16H), 2.36 (ddd, J=9.8, 6.4, 1.9Hz, 1H), 2.70-2.89 -(m, 2H), 3.59 (s, 2H), 3.95-4.05 (m, 1H), 4.15 (q, J=7.2Hz, 2H), 4.32-4.45,(m, 1H), 4.39 (dt, J=1.9, 7.0HZ, 1H), 7.07-7.30 (m, 4H)
   IR (neat) : 3400, 2945, 2834, 2285, 1734, 1608, 1590, 1489, 1446, 1385, 1369, 1300, 1256, 1152, 1093, 1032, 787, 700cm⁻¹

### Example 4

### 9-Deoxy-9β-chloro-3, 4, 5, 17, 18, 19, 20-heptanol-2, 6-inter-m-phenylene-16-cyclopentyl-13, 14-didehydro-PGF₁α (Compound 70)

Following the substantially same manner as in Example 2 using the compound obtained in Example 3(5), thereby the title compound was obtained.
¹H-NMR(CDCl₃, 300MHz) δ ppm; 1.03-1.20 (m, 2H), 1.41-2.37 (m, 16H), 2.32 (ddd, J=9.8, 6.6, 2.0Hz, 1H), 2.74-2.86 (m, 2H), 3.62 (s, 2H), 3.95-4.05 (m, 1H), 4.28-4.45 (m, 3H), 7.07-7.31 (m, 4H)
IR(neat): 3368, 2946, 2864, 2236, 1712, 1609, 1590, 1490, 1449, 1409, 1327, 1251, 1158, 1085, 1038, 936, 762, 704, 614 cm⁻¹

### Example 5

### 9-Deoxy-9β-chloro-3, 4, 5, 16, 17, 18, 19, 20-octanol-2, 6-inter-m-phenylene-15-cycloheptyl-13, 14-didehydro-PGF₁α ethyl ester (Compound 59)

(1) Following the substantially same manner as in Example 1(1) using
   (3S)-3-(tert-butyldimethylsiloxy)-3-cycloheptylprop-1-yne in place of
   (3S)-3-(tert-butyldimethylsiloxy)-3-cyclohexylprop-1 -yne in Example 1(1), thereby (3R, 4R)-2-methylene-3-[(3S)-3-(tert-butyldimethylsiloxy) -3-cycloheptylprop-1-ynyl]-4-(tert-butyldimethylsilo xy)cyclopentane-1-one was obtained.
   ¹H-NMR (CDCl₃ 200MHz) δ ppm; 0.08 (s, 3H), 0.10 (s, 6H), 0.13 (s, 3H), 0.89 (s, 9H), 0.90 (s, 9H), 1.16-1.93 (m, 13H), 2.33 (dd, J=17.9, 7.4Hz, 1H), 2.76 (dd, J=17.9, 6.5Hz, 1H), 3.49-3.57 (m, 1H), 4.14-4.22 (m, 2H), 5.56 (d, J=2.6Hz, 1H), 6.14 (d, J=2.9Hz, 1H)
   IR(neat) : 2929, 2857, 2233, 1737, 1645, 1463, 1389, 1362, 1253, 1223, 1123, 1087, 1007, 941, 838, 778, 670 cm⁻¹
(2) Following the substantially same manner as in Example 1(2) using the compound obtained in (1) above, thereby 3, 4, 5, 16, 17, 18, 19, 20-octanol-2, 6-inter-m-phenylene-15-cycloheptyl-13, 14-didehydro-PGE₁ ethyl ester 11, 15-bis(tert-butyldimethylsilyl ether) was obtained.
   ¹H-NMR(CDCl₃ 300MHz) δ ppm; 0.08 (s, 3H), 0.10 (s, 6H), 0.14 (s, 3H), 0.89 (s, 9H), 0.90 (s, 9H), 1.16-2.23 (m, 16H), 1.25 (t, J=7.2Hz, 3H), 2.20 (dd, J=18.3, 6.5Hz, 1H), 2.63-2.85 (m, 4H), 3.57 (s, 2H), 4.10.-4.23 (m, 1H), 4.15 (q, J=7.2Hz, 2H), 4.26-4.36 (m, 1H), 7.05-7.31 (m, 4H)
   IR(neat) : 2929, 2857, 2236, 1746, 1609, 1472, 1463, 1368, 1338, 1252, 1149, 1085, 1034, 1006, 939, 884, 838, 778, 700, 670 cm⁻¹
(3) Following the substantially same manner as in Example 1(3) using the compound obtained in (2) above, thereby 3, 4, 5, 16, 17, 18, 19, 20-octanol-2, 6-inter-m-phenylene-15-cycloheptyl-13, 14-didehydro-PGF₁α ethyl ester 11, 15-bis(tert-butyldimethylsilyl ether) was obtained.
   ¹H-NMR (CDCl₃, 200MHz) δ ppm; 0.07 (s, 3H), 0.09 (s, 6H), 0.11 (s, 3H), 0.88 (s, 9H), 0.89 (s, 9H), 1.18-2.08 (m, 18H), 1.25 (t, J=7.1Hz, 3H), 2.43-2.90 (m, 4H), 3.58 (s, 2H), 4.08-4.34 (m, 3H), 4.15 (q, J=7.1Hz, 2H), 7.06-7.34 (m, 4H)
   IR (neat) : 3468, 2929, 2857, 2230, 1739, 1609, 1472, 1463, 1386, 1362, 1338, 1252, 1131, 1084, 1035, 1006, 884, 837, 777, 700, 668 cm⁻¹
(4) Following the substantially same manner as in Example 1(4) using the compound obtained in (3) above, thereby 9-deoxy-9β-chloro-3, 4, 5, 16, 17, 18, 19, 20-octanol-2, 6-inter-m-phenylene-15-cycloheptyl-13, 14-didehydro-PGF₁α ethyl ester 11, 15-bis(tert-butyldimethylsilyl ether) was obtained.
   ¹H-NMR(CDCl₃, 300MHz) δ ppm; 0.07 (s, 3H), 0.08 (s, 3H), 0.10 (s, 3H), 0.11 (s, 3H), 0.88 (s, 9H), 0.89 (s, 9H), 1.21-2.21 (m, 18H), 1.26 (t, J=7.2Hz, 3H), 2.33-2.41 (m, 1H), 2.66-2.85 (m, 2H), 3.59 (s, 2H), 3.96-4.06 (m, 1H), 4.10-4.22 (m, 1H), 4.15 (q, J=7.2Hz, 2H), 4.23-4.31 (m, 1H), 7.08-7.16 (m, 3H), 7.21-7.28 (m, 1H)
   IR (neat) : 2929, 2857, 2231, 1740, 1609, 1472, 1463, 1363, 1338, 1252, 1150, 1085, 1035, 1006, 940, 837, 777, 700, 600 cm⁻¹
(5) Following the substantially same manner as in Example 1(5) using the compound obtained in (4) above, thereby the title compound was obtained.
   ¹H-NMR (CDCl₃, 300MHz) δ ppm; 1.19-2.41 (m, 20H), 1.26 (t, J=7.2Hz, 3H), 2.36 (ddd, J=9.7, 6.3, 1.9Hz, 1H), 2.70-2.90 (m, 2H), 3.59 (s, 2H), 3.96-4.05 (m, 1H), 4.15 (q, J=7.2Hz, 2H), 4.23 (dd, J=5.3, 1.9Hz, 1H), 4.32-4.42 (m, 1H), 7.07=7.30 (m, 4H)
   IR(neat): 3400, 2980, 2924, 2855, 2233, 1734, 1608, 1590, 1446, 1369, 1333, 1300, 1256, 1151, 1066, 1031, 786, 700 cm⁻¹

### Example 6

### 9-Deoxy-9β-chloro-3, 4, 5, 16, 17, 18, 19, 20-octanol-2, 6-inter-m-phenylene-15-cycloheptyl-13, 14-didehydro-PGF₁α (Compound 60)

Following the substantially same manner as in Example 2 using the compound obtained in Example 5(5), thereby the title compound was obtained.
¹H-NMR (CDCl₃, 300MHz) δ ppm: 1.22-2.38 (m, 22H), 2.69-2.90 (m, 2H), 3.63 (s, 2H), 3.95-4.06 (m 1H), 4.24 (dd/J=5.3, 1.9Hz, 1H), 4.30-4.39 (m, 1H), 7.07-7.30 (m, 4H)
IR(neat) : 3368, 2924, 2856, 2679, 2235, 1712, 1609, 1590, 1489, 1446, 1409, 1385, 1329, 1251, 1154, 1047, 1014, 987, 760, 703, 616 cm⁻¹

### Example 7

### 9-Deoxy-9β-chloro-3, 4, 5, 16, 17, 18, 19, 20-octanol-2, 6-inter-m-phenylene-15-cyclopentyl-13, 14-didehydro-PGF₁α ethyl ester (Compound 32)

(1) Following the substantially same manner as in Example 1(1) using
   (3S)-3-(tert-butyldimethylsiloxy)-3-cyclopentylprop-1-yne in place of
   (3S)-3-(tert-butyldimethylsiloxy)-3-cyclohexylprop-1 -yne in Example 1(1), thereby (3R, 4R)-2-methylene-3-[(3S)-3-(tert-butyldimethylsiloxy) -3-cyclopentylprop-1-ynyl]-4-(tert-butyldimethylsilo xy)cyclopentane-1-one was obtained.
   ¹H-NMR(CDCl₃, 200MHz) δppm; 0.09 (s, 3H), 0.10 (s, 6H), 0.13 (s, 3H), 0.89 (s, 9H), 0.90 (s, 9H), 1.18-1.80 (m, 8H), 2.0.6-2.24, (m, 1H), 2.32 (dd, J=17.9, 7.3Hz, 1H), 2.72 (dd, J=17.9, 6.4Hz, 1H), 3.48-3.57 (m, 1H), 4.16-4.31 (m, 2H), 5.54 (dd, J=2.6, 0.7Hz, 1H), 6.14 (dd, J=2.9, 0.7Hz, 1H)
   IR(neat): 2955, 2931, 2858, 2234, 1734, 1646, 1473, 1463, 1388, 1362, 1255, 1222, 1099, 838, 778, 671 cm⁻¹
(2) Following the substantially same manner as in Example 1(2) using the compound obtained in (1) above, thereby 3, 4, 5, 16, 17, 18, 19, 20-octanol-2, 6-inter-m-phenylene-15-cyclopentyl-13, 14-didehydro-PGE₁ ethyl ester 11, 15-bis(tert-butyldimethylsilyl ether) was obtained.
   ¹H-NMR(CDCl₃, 300MHz) *δ*ppm; 0.08 (s, 3H), 0.10 (s, 3H), 0.11 (s, 3H), 0.13 (s, 3H), 0.89 (s, 18H), 1.19-2.29 (m, 12H), 1.25 (t, J=7.2Hz, 3H), 2.20 (dd, J=18.2, 6.7Hz, 1H) 2.64-2.83 (m, 4H), 3.57 (s, 2H), 4.15 (q, J=7.2Hz, 2H) 4.19 (dd, J=6.8, 1.4Hz, 1H), 4.26-4.35(m, 1H), 7.06-7.27 (m, 4H)
   IR(neat) : 2955, 2930, 2858, 2236, 1746, 1609, 1472, 1363, 1329, 1252, 1150, 1096, 1035, 1006, 940, 887, 838, 778, 700, 670 cm⁻¹
(3) Following the substantially same manner as in Example 1(3) using the compound obtained in (2) above, thereby 3, 4, 5, 16, 17, 18, 19, 20-octanol-2, 6-inter-m-phenylene-15-cyclopentyl-13, 14-didehydro-PGF₁α ethyl ester 11, 15-bis(tert-butyldimethylsilyl ether) was obtained.
   ¹H-NMR (CDCl₃, 300MHz) δ ppm; 0.08 (s, 3H), 0.09 (s, 3H), 0.10 (s, 3H), 0.11 (s, 3H), 0.88 (s, 9H), 0.89 (s, 9H), 1.18-2.18, (m, 14H), 1.25 (t, J=7.2Hz, 3H), 2.42-2.85 (m, 4H), 3.58 (s, 2H), 4.09-4.20 (m, 1H), 4.15 (q, J=7.2Hz, 2H), 4.16 (dd, J=7.0, 1.9Hz, 1H), 4.24-4.30 (m, 1H), 7.07-7.27 (m, 4H)
   IR(neat) : 3468, 2955, 2930, 2858, 2231, 1739, 1609, 1472, 1386, 1362, 1327, 1253, 1100, 1071, 1035, 1006, 940, 838, 777, 701, 670 cm⁻¹
(4) Following the substantially same manner as in Example 1(4) using the compound obtained in (3) above, thereby 9-deoxy-9β-chloro-3, 4, 5, 16, 17, 18, 19, 20-octanol-2, 6-inter-m-phenylene-15-cyclopentyl-13, 14-didehydro-PGF₁α ethyl ester 11, 15-bis(tert-butyldimethylsilyl ether) was obtained.
   ¹H-NMR(CDCl₃, 300MHz) δ ppm; 0.08 (s, 3H), 0.09 (s, 3H), 0.10 (s, 3H), 0.12 (s, 3H), 0.88 (s, 9H), 0.90 (s, 9H), 1.19-2.20 (m, 14H), 1.26 (t, = J=7.2Hz, 3H), 2.36 (ddd; J=8.7, 4.8, 1.7Hz, 1H), 2.66-2.86 (m, 2H), 3.59 (s, 2H), 3.95-4.06 (m, 1H), 4.15 (q, J=7.2Hz, 2H), 4.18 (dd, J=6.8, 1.7Hz, 1H), 4.22-4.30 (m, 1H), 7.06-7.28 (m, 4H)
   IR(neat) : 2955, 2930, 2858, 2232, 1740, 1609, 1472, 1463, 1386, 1363, 1329, 1253, 1151, 1096, 1035, 1006, 940, 904, 837, 777, 700, 670 cm⁻¹
(5) Following the substantially same manner as in Example 1(5) using the compound obtained in (4) above, thereby the title compound was obtained.
   ¹H-NMR(CDCl₃, 300MHz) δ ppm; 1.26 (t, J=7.2Hz 3H), 1.29-2.48 (m, 17H), 2.70-2.88 (m, 2H), 3.59 (s, 2H), 3.95-4.05 (m, 1H), 4.15 (q, J=7.2Hz, 2H), 4.21-4.28 (m, 1H), 4.31-4.41 (m, 1H), 7.03-7.29 (m, 4H)
   IR(neat) : 3400, 2952, 2866, 2235, 1734, 1608, 1590, 1489, 1447, 1369, 1300, 1256, 1152, 1093, 1029, 940, 774, 701, 638 cm⁻¹

### Example 8

### 9-Deoxy-9β-chloro-3, 4, 5, 16, 17, 18, 19, 20-octanol-2, 6-inter-m-phenylene-15-cyclopentyl-13, 14-didehydro-PGF₁α (Compound 33)

Following the substantially same manner as in Example 2 using the compound obtained in Example 7(5), thereby the title compound was obtained.
¹H-NMR (CDCl₃, 300MHz) δ ppm; 1.21-2.89 (m, 19H), 2.33 (ddd, J=9.7, 6.7, 1.8Hz, 1H), 3.62 (s, 2H), 3.95-4.05 (m, 1H), 4.25 (dd, J=7.1, 1.8Hz, 1H), 4.29-4.38 (m, 1H), 7.05-7.30 (m, 4H)
IR (neat) : 3368, 2952, 2866, 2236, 1712, 1609, 1590, 1489, 1447, 1408, 1385, 1322, 1251, 1154, 1084, 1052, 1025, 939, 762, 704, 614 cm⁻¹

### Example 9

### 9-Deoxy-9β-chloro-3, 4, 5, 17, 18, 19, 20-heptanol-2, 6-inter-m-phenylene-16-cyclohexyl-13, 14-didehydro-PGF₁α ethyl ester (Compound 71)

(1) Following the substantially same manner as in Example 1(1) using
   (3S)-3-(tert-butyldimethylsiloxy)-4-cyclohexylbut-1-yne in place of
   (3S)-3-(tert-butyldimethylsiloxy)-3-cyclohexylprop-1 -yne in Example 1(1), thereby (3R, 4R)-2-methylene-3-[(3S)-3-(tert-butyldimethylsiloxy) -4-cyclohexylbut-1-ynyl]-4-(tert-butyldimethylsiloxy) cyclopentane-1-one was obtained.
   ¹H-NMR(CDCl₃, 200MXz) δ ppm; 0.10 (s, 3H), 0.11 (s, 6H), 0.14 (s, 3H), 0.70-1.78 (m, 13H), 0.90 (s, 18H), 2.33 (dd, J=18.0, 7.4Hz, 1H), 2.72 (dd, J=18.0, 6.5Hz, 1H), 3.46-3.57 (m, 1H), 4.21-4.33 (m, 1H), 4.48 (dt, J=1.5, 7.4Hz, 1H), 5.56 (d, J=2.6Hz, 1H), 6.14 (d, J=3.0Hz, 1H)
   IR (neat) : 2927, 2857, 2233, 1738, 1646, 1473, 1463, 1389, 1362, 1255, 1098, 1005, 939, 839, 778, 670, 575 cm⁻¹
(2) Following the substantially same manner as in Example 1(2) using the compound obtained in (1) above, thereby 3, 4, 5, 17, 18, 19, 20-heptanol-2, 6-inter-m-phenylene-16-cyclohexyl-13, 14-didehydro-PGE₁ ethyl ester 11, 15-bis(tert-butyldimethylsilyl ether) was obtained.
   ¹H-NMR(CDCl₃, 300MHz) δ ppm; 0.09 (s, 3H), 0.10 (s, 3H), 0.11 (s, 3H), 0.14 (s, 3H) 0.75-2.31 (m, 16H), 0.89 (s, 9H), 0.90 (s, 9H), 1.25 (t, J=7.2Hz, 3H), 2.20 (dd, J=18.3, 6.9Hz, 1H), 2.63-2.85 (m, 4H), 3.57 (s, 2H), 4.15 (q, J=7.2Hz, 2H), 4.25-4.36 (m, 1H), 4.39-4.49 (m, 1H), 7.06-7.28 (m, 4H)
   IR(neat) : 2928, 2856, 2236, 1746, 1609, 1472, 1463, 1448, 1368, 1252, 1149, 1096, 1075, 1034, 1004, 938, 884, 838, 778, 700, 670 cm⁻¹
(3) Following the substantially same manner as in Example 1(3) using the compound obtained in (2) above, thereby 3, 4, 5, 17, 18, 19, 20-heptanol-2, 6-inter-m-phenylene-16-cyclohexyl-13, 14-didehydro-PGF₁α ethyl ester 11, 15-bis(tert-butyldimethylsilyl ether) was obtained.
   ¹H-NMR (CDCl₃, 300MHz) δ ppm; 0.09 (s, 6H), 0.10 (s, 3H) 0.11 (s, 3H), 0.76-2.07 (m, 18H), 0.89 (s, 9H), 0.90 (s, 9H), 1.25 (t, J=7.2Hz, 3H), 2.41-2.85 (m, 3H), 2.50 (d, J=8.7Hz, 1H), 3.58 (s, 2H), 4.09-4.19 (m, 1H), 4.15 (q, J=7.2Hz, 2H), 4.25-4.31 (m, 1H), 4.39-4.46 (m, 1H), 7.07=7.27 (m, 4H)
   IR(neat): 3468, 2928, 2855, 2231, 1740, 1609, 1472, 1463 1448, 1387 1362, 1337, 1253, 1131, 1096, 1074, 1004, 939, 838, 777, 668 cm⁻¹
(4) Following the substantially same manner as in Example 1(4) using the compound obtained in (3) above, thereby 9-deoxy-9β-chloro-3, 4, 5, 17, 18, 19, 20-heptanol-2, 6-inter-m-phenylene-16-cyclohexyl-13, 14-didehydro-PGF₁α ethyl ester 11, 15-bis(tert-butyldimethylsilyl ether) was obtained.
   ¹H-NMR(CDCl₃, 300MHz) δ ppm; 0.09 (s, 3H), 0.10 (s, 6H), 0.12 (s, 3H), 0.76-2.23 (m, 18H), 0.89 (s, 9H), 0.90 (s, 9H), 1.26 (t, J=7.2Hz, 3H), 2.31-2.40 (m, 1H), 2.66-2.85 (m, 2H, 3.59 (s, 2H), 3.96-4.06 (m, 1H), 4.15 (q, J=7.2Hz, 2H), 4.23-4.31 (m, 1H), 4.40-4.48 (m, 1H), 7.06-7.29, (m, 4H)
   IR(neat) : 2928, 2856, 2232, 1740, 1609, 1472, 1463, 1448, 1388, 1363, 1253, 1151, 1096, 1074, 1035, 1004, 939, 837, 810, 778, 700, 669 cm⁻¹
(5) Following the substantially same manner as in Example 1(5) using the compound obtained in (4) above, thereby the title compound was obtained.
   ¹H-NMR(CDCl₃, 300MHz) δ ppm; 0.84-2.40 (m, 20H), 1.26 (t, J=7.2Hz, 3H), 2.36 (ddd, J=9.8, 6.3, 1.8Hz, 1H), 2.70-2.89 (m, 2H), 3.59 (s, 2H), 3.95-4.05 (m, 1H), 4.15 (q, J=7.2Hz, 2H), 4.31-4.50 (m, 2H), 7.06-7.29 (m, 4H)
   IR (neat) : 3400, 2980, 2924, .2851, 2238, 1734, 1608, 1590, 1489, 1448, 1385, 1369, 1334, 1299, 1257, 1152, 1089, 1031, 894, 774, 700 cm⁻¹

### Example 10

### 9-Deoxy-9β-chloro-3, 4, 5, 17, 18, 19, 20-heptanol-2, 6-inter-m-phenylene-16-cyclohexyl-13, 14-didehydro-PGF₁α (Compound 72)

Following the substantially same manner as in Example 2 using the compound obtained in Example 9(5), thereby the title compound was obtained.
¹H-NMR(CDCl₃, 300MHz), δppm; 0.81-2.39 (m, 18H), 2.32 (ddd, J=9.8, 6.6, 1.7Hz, 1H), 2.71-2.89 (m, 2H), 3.63 (s, 2H), 3.82-4.52 (m, 6H), 7.05-7.30 (m, 4H)
IR(neat) : 3368, 2924, 2851, 2236, 1712, 1609, 1490, 1448, 1411, 1266, 1155, 1085, 1030, 981, 761, 704, 615 cm⁻¹

### Example 11

### 9-Deoxy-9β-chloro-3, 4, 5, 16, 17, 18, 19, 20-octanol-2, 6-inter-m-phenylene-15-cyclooctyl-13, 14-didehydro-PGF₁α ethyl ester (Compound 65)

(1) Following the substantially same manner as in Example 1(1) using
   (3S)-3-(tert-butyldimethylsiloxy)-3-cyclooctylprop-1 -yne in place of
   (3S)-3-(tert-butyldimethylsiloxy)-3-cyclohexylprop-1 -yne in Example 1(1), thereby (3R, 4R)-2-methylene-3-[(3S)-3-(tert-butyldimethylsiloxy) -3-cyclooctylprop-1-ynyl]-4-(tert-butyldimethylsilox y)cyclopentane-1-one was obtained.
   ¹H-NMR(CDCl₃, 300MHz) δ ppm; 0.08 (s, 3H), 0.10 (2s, 6H), 0.13 (s, 3H), 0.89 (s, 9H), 0.90 (s, 9H), 1.18-1.84 (m, 15H), 2.33 (dd, J=17.9, 7.3Hz, 1H), 2.72 (dd, J=17.9, 6.5Hz, 1H), 3.49-3.56 (m, 1H), 4.16 (dd, J=5.3, 1.6Hz, 1H), 4.21-4.31 (m, 1H), 5.56 (d, J=2.6Hz, 1H), 6.14 (d, J=3.0Hz, 1H)
   IR(neat): 2928, 2857, 2362, 1737, 1646, 1472, 1386, 1252, 1222, 1121, 1078, 1006, 941, 837, 777, 670 cm⁻¹
(2) Following the substantially same manner as in Example 1(2) using the compound obtained in (1) above, thereby 3, 4, 5, 16, 17, 18, 19, 20-octanol-2, 6-inter-m-phenylene-15-cyclooctyl-13, 14-didehydro-PGE₁ ethyl ester 11, 15-bis(tert-butyldimethylsilyl ether) was obtained.
   ¹H-NMR(CDCl₃, 300MHz) δ ppm; 0.08 (s, 3H), 0.10 (2s, 6H), 0.14 (s, 3H), 0.89 (s, 9H), 0.90 (s, 9H), 1.16-2.35 (m, 18H), 1.25 (t, J=7.2Hz, 3H), 2.20 (dd, J=18.3, 6.5Hz, 1H), 2.62-2.84 (m, 4H), 3.58 (s, 2H), 4.09-4.20 (m, 1H), 4.15 (q, J=7.2Hz, 2H), 4.26-4.35 (m, 1H), 7.04-7.28 (m, 4H)
   IR(neat) : 2928, 2857, 2236, 1746, 1447, 1368, 1337, 1252, 1149, 1094, 1034, 1006, 940, 837, 778, 700, 670 cm⁻¹
(3) Following the substantially same manner as in Example 1(3) using the compound obtained in (2) above, thereby 3, 4, 5, 16, 17, 18, 19, 20-octanol-2, 6-inter-m-phenylene-15-cyclooctyl-13, 14-didehydro-PGF₁α ethyl ester 11, 15-bis(tert-butyldimethylsilyl ether) was obtained.
   ¹H-NMR (CDCl₃, 300MHz) δ ppm; 0.07 (s, 3H), 0.09 (s, 6H), 0.11 (s, 3H), 0.88 (s, 9H), 0.89 (s, 9H), 1.20-2.05 (m, 20H), 1.25 (t, J=7.2Hz, 3H), 2.41-2.85 (m, 4H), 3.58 (s, 2H), 4.06-4.20 (m, 1H), 4.10 (dd, J=5.4, 1:7Hz, 1H), 4.15 (q, J=7.2Hz, 2H), 4.25-4.31 (m, 1H), 7.07-7.27 (m, 4H)
   IR(neat) : 3524, 2929, 2857, 2230, 1740, 1609, 1472, 1446, 1388, 1362, 1338, 1253, 1132, 1078, 1035, 1006, 940, 837, 778, 701, 670cm⁻¹
(4) Following the substantially same manner as in Example 1 (4) using the compound obtained in (3) above, thereby 9-deoxy-9β-chloro-3, 4, 5, 16, 17, 18, 19, 20-octanol-2, 6-inter-m-phenylene-15-cyclooctyl-13, 14-didehydro-PGF₁α ethyl ester 11, 15-bis(tert-butyldimethylsilyl ether) was obtained.
   ¹H-NMR (CDCl₃, 300MHz) δ ppm; 0.08 (2s, 6H), 0.10 (s, 3H), 0.12 (s, 3H), 0.89 (s, 9H), 0.90 (s, 9H) 1.21-2.20 (m, 20H), 1.26 (t, J=7.2Hz, 3H), 2.32-2.40 (m, 1H), 2.66-2.86 (m, 2H), 3.59 (s, 2H), 3.96-4.06 (m, 1H), 4.08-4.20 (m, 1H), 4.15 (q, J=7.2Hz, 2H), 4.23-4.30 (m, 1H), 7.06-7.29 (m, 4H)
   IR(neat): 2928, 2857, 2231, 1740, 1609, 1472, 1446, 1388, 1363, 1338, 1253, 1151, 1082, 1035, 1006, 940, 837, 813, 778, 700, 670 cm⁻¹
(5) Following the substantially same manner as in Example 1(5) using the compound obtained in (4) above, thereby the title compound was obtained.
   ¹H-NMR (CDCl₃ 300MHz) δ ppm; 1.12-2.40 (m, 22H), 1.26 (t, J=7.2Hz, 3H), 2.35, (ddd, J=9.7, 6.6, 1.9Hz, 1H), 2.69-2.89 (m, 2H), 3.59 (s, 2H), 3.95-4.05 (m, 1H), 4.07-4.23 (m, 1H), 4.15 (q, J=7.2Hz, 2H, 4.30-4.46 (m, 1H), 7.07-7.29 (m, 4H)
   IR(neat): 3400, 2919, 2855, 2234, 1734, 1608, 1446, 1385, 1369, 1333, 1300, 1256, 1151, 1030 774, 700 cm⁻¹

### Example 12

### 9-Deoxy-9β-chloro-3, 4, 5, 16, 17, 18, 19, 20-octanol-2, 6-inter-m-phenylene-15-cyclooctyl-13, 14-didehydro-PGF₁α (Compound 66)

Following the substantially same manner as in Example 2 using the compound obtained in Example 11(5), thereby the title compound was obtained.
¹H-NMR (CDCl₃, 300MHz) δ ppm; 1.20-2.39 (m, 20H), 2.33 (ddd, J=9.6, 6.5, 1.8Hz, 1H), 2.71-2.89 (m, 2H), 3.63 (s, 2H), 3.70-4.40 (m, 5H), 4.20 (dd, J=5.4, 1.8Hz, 1H), 7.05-7.30 (m, 4H)
IR (neat) : 3368, 2921, 2856, 2235, 1712, 1609, 1590, 1489, 1446, 1408, 1252, 1154, 1085, 1025, 759, 704, 614 cm⁻¹

### Example 13

### (15R)-9-Deoxy-9β-chloro-3, 4, 5, 16, 17, 18, 19, 20-octanol-2, 6-inter-m-phenylene-15-cyclohexyl-13, 14-didehydro-PGF₁α ethyl ester (Compound 39)

(1) Following the substantially same manner as in Example 1(1) using
   (3R)-3-(tert-butyldimethylsiloxy)-3-cyclohexylprop-1 -yne in place of
   (3S)-3-(tert-butyldimethylsiloxy)-3-cyclohexylprop-1 -yne in Example 1(1), thereby (3R, 4R)-2-methylene-3-[(3R)-3-(tert-butyldimethylsiloxy) -3-cyclohexylprop-1-ynyl]-4-(tert-butyldimethylsilox y)cyclopentane-1-one was obtained.
   ¹H-NMR (CDCl₃, 300MHz) δ ppm; 0.10 (s, 6H), 0.13 (s, 6H), 0.82-1.92 (m, 11H), 0.90 (s, 9H), 0.91 (s, 9H) 2.33 (dd, J=17.9, 7.3Hz, 1H), 2.72 (dd, J=17.9, 6.5Hz, 1H), 3.50-3.59 (m, 1H), 4.06-4.15 (m, 1H), 4.22-4.32 (m, 1H), 5.56 (d, J=2.5Hz, 1H), 6.14 (d, J=3.1Hz, 1H)
   IR(neat) : 2930, 2856, 2235, 1737, 1645, 1473, 1451, 1388, 1253, 1233, 1109, 1007, 941, 899, 838, 778, 670 cm⁻¹
(2) Following the substantially same manner as in Example 1(2) using the compound obtained in (1) above, thereby (15R)-3, 4, 5, 16, 17, 18, 19, 20-octanol-2, 6-inter-m-phenylene-15-cyclohexyl-13, 14-didehydro-PGE₁ ethyl ester 11, 15-bis(tert-butyldimethylsilyl ether) was obtained.
   ¹H-NMR (CDCl₃, 300MHz) δ ppm; 0.08 (s, 3H), 0.09 (s, 3H), 0.10 (s, 3H), 0.13 (s, 3H), 0.90 (s, 18H), 0.82-2.12 (m, 13H), 1.25 (t, J=7.2Hz, 3H), 2.20 (dd, J=18.3, 6.6Hz, 1H), 2.20-2.30 (m, 1H), 2.63-2.85 (m, 4H), 3.57 (s, 2H), 4.10 (dd, J=6.1, 1.4Hz, 1H), 4.15 (q, J=7.2Hz, 2H), 4.26-4.36 (m, 1H), 7.06-7.27 (m, 4H)
   IR(neat):3228, 2929, 2856, 2360, 1746, 1630, 1472, 1463, 1450, 1369, 1337, 1294, 1252, 1100, 1069, 1035, 1006, 898, 883, 838, 778, 700, 670, 596 cm⁻¹
(3) Following the substantially same manner as in Example 1(3) using the compound obtained in (2) above, thereby (15R)-3, 4, 5, 16, 17, 18, 19, 20-octanol-2, 6-inter-m-phenylene-15-cyclohexyl-13, 14-didehydro-PGF₁α ethyl ester 11, 15-bis(tert-butyldimethylsilyl ether) was obtained.
   ¹H-NMR (CDCl₃ 300MHz) δ ppm; 0.08 (s, 3H), 0.09 (s, 3H), 0.10 (s, 3H) 0.11 (s, 3H), 0.80-2.08 (m, 16H), 0.88 (s, 9H), 0.90 (s, 9H), 1.25 (t, J=7.2Hz, 3H), 2.44-2.87 (m, 4H), 3.58 (s, 2H), 4.07 (dd, J=6.4, 2.0Xz, 1H), 4.10-4.20 (m, 1H), 4.15 (q, J=7.2Hz, 2H), 4.26-4.31 (m, 1H), 7.07-7.30 (m, 4H)
   IR(neat): 3524, 2930, 2855, 2232, 1740, 1609, 1472, 1450, 1389, 1368, 1337, 1256, 1100, 1006, 963, 940, 898, 837, 777, 701, 668 cm⁻¹
(4) Following the substantially same manner as in Example 1(4) using the compound obtained in (3) above, thereby (15R)-9-deoxy-9β-chloro-3, 4, 5, 16, 17, 18, 19, 20-octanol-2, 6-inter-m-phenylene-15-cyclohexyl-13, 14-didehydro-PGF₁α ethyl ester 11, 15-bis(tert-butyldimethylsilyl ether) was obtained.
   ¹H-NMR (CDCl₃, 300MHz) δ ppm; 0.08 (s, 6H), 0.09 (s, 3H), 0.11 (s 3H), 0.78-2.06 (m, 13H), 0.88 (s, 9H), 0.90 (s, 9H), 1.25 (t, J=7.2Hz, 3H), 2.09-2.24 (m, 3H), 2.37 (ddd, J=8.9, 4.8, 1.7Hz, 1H), 2.63-2.86 (m, 2H), 3.58(s, 2H), 3.96-4.06 (m, 1H), 4.09 (dd, J=6.2, 1.7Hz, 1H), 4.15 (q, J=7.2Hz, 2H), 4.24-4.32 (m, 1H), 7.07-7.28 (m, 4H)
   IR(neat) : 2930, 2855, 2233, 1740, 1609, 1472, 1463, 1450, 1389, 1367, 1337, 1252, 1150, 1100, 1034, 1006, 939, 898, 837, 778, 700, 670 cm⁻¹
(5) Following the substantially same manner as in Example 1(5) using the compound obtained in (4) above, thereby the title compound was obtained.
   ¹H-NMR (CDCl₃, 300MHz) δ ppm; 0.95-2.41 (m, 18H), 1.26 (t, J=7.2Hz, 3H), 2.37 (ddd, J=9.8, 6.4, 1.7Hz, 1H), 2.70-2.89 (m, 2H), 3.59 (s, 2H), 3.96-4.06 (m, 1H), 4.15 (q, J=7.2Hz, 2H), 4.08-4.20 (m, 1H), 4.32-4.43 (m, 1H), 7.07-7.28 (m, 4H)
   IR(neat):3400, 2928, 2854, 2236, 1734, 1608, 1590, 1489, 1450, 1396, 1370, 1335, 1259, 1153, 1084, 1030, 957, 893, 774, 701, 578cm⁻¹

### Example 14

### (15R)-9-Deoxy-9β-chloro-3, 4, 5, 16, 17, 18, 19, 20-octanol-2, 6-inter-m-phenylene-15-cyclohexyl-13, 14-didehydro-PGF₁α (Compound 40)

Following the substantially same manner as in Example 2 using the compound obtained in Example 13(5), thereby the title compound was obtained.
¹H=NMR(CDCl₃, 300MHz) δ ppm; 0.94-1.36 (m, 5H), 1.44-2.03 (m, 11H), 2.12-2.38 (m, 3H), 2.34 (ddd, J=9.6, 6.5, 1.8Hz), 2.74-2.90 (m, 2H), 3.63 (s, 2H), 3.96-4.07 (m, 1H), 4.16 (dd, J=6.1, 1.8Hz, 1H), 4.30-4.42 (m, 1H), 7.08-7.30 (m, 4H)
IR (neat) : 3368, 2929, 2854, 2236, 1712, 1609, 1590, 1490, 1450, 1407, 1262, 1154, 1084, 1008, 957, 894, 759, 704, 614 cm⁻¹

### Example 15

### 9-Deoxy-9α-chloro-3, 4, 5, 16, 17, 18, 19, 20-octanol-2, 6-inter-m-phenylene-15-cyclohexyl-13, 14-didehydro-PGF₁β ethyl ester (Compound 46)

(1) Following the substantially same manner as in Example 1(4) using 3, 4, 5, 16, 17, 18, 19, 20-octanol-2, 6-inter-m-phenylene-15-cyclohexyl-13, 14-didehydro-PGF₁β ethyl ester 11, 15-bis(tert-butyldimethylsilyl ether) obtained in Example 1(3), 9-deoxy-9α-chloro-3, 4, 5, 16, 17, 18, 19, 20-octanol-2, 6-inter-m-phenylene-15-cyclohexyl-13, 14-didehydro-PGF₁β ethyl ester 11, 15-bis(tert-butyldimethylsilyl ether) was obtained.
   ¹H-NMR(CDCl₃, 300MHz) δppm; 0:06 (s, 3H), 0.07 (s, 3H), 0.09 (2s, 6H), 0.82-2.19 (m, 15H), 0.89 (2s, 18H), 1.25 (t, J=7.2Hz, 3H), 2.48-2.83 (m, 4H), 3.58 (s, 2H), 4.08 (dd, J=6.1, 1.4Hz, 1H), 4.11-4.22 (m, 1H), 4.15 (q, J=7.2Hz, 2H), 4.33-4.39 (m, 1H), 7.08-7.28 (m, 4H)
   IR(neat) : 2929, 2856, 2235, 1740, 1609, 1472, 1463, 1450, 1368, 1337, 1252, 1100, 1068, 1034, 1006, 939, 888, 837, 777, 700, 668 cm⁻¹
(2) Following the substantially same manner as in Example 1(5) using the compound obtained in (1) above, thereby the title compound was obtained.
   ¹H-NMR(CDCl₃, 300MHz) δppm; 0.97-2.32 (m, 17H), 1.26 (t, J=7.2Hz, 3H), 2.53-2.90 (m, 4H), 3.59 (s, 2H), 4.08-4.30 (m, 2H), 4.15 (q,J=7.2Hz, 2H), 4.38-4.44 (m, 1H), 7.08-7.17 (m, 2H), 7.20-7.28 (m, 2H)
   IR(neat) : 3400, 2928, 2853, 2236, 1734, 1608, 1489, 1450, 1369, 1257, 1153, 1084, 1031, 893, 834, 773, 700, 619 cm⁻¹

### Example 16

### 9-Deoxy-9α-chloro-3, 4, 5, 16, 17, 18, 19, 20-octanol-2, 6-inter-m-phenylene-15-cyclohexyl-13, 14-didehydro-PGF₁β (Compound 47)

Following the substantially same manner as in Example 2 using the compound obtained in Example 15(2), thereby the title compound was obtained.
¹H-NMR (CDCl₃, 300MHz) δ ppm ; 0.85-2.17 (m, 18H), 2.48-2.65 (m, 2H), 2.74 (ddd, 1=11.4, 6.1, 1.7Hz, 1H), 2.79-2.93 (m, 1H), 3.58 (d, J=15.1Hz, 1H), 3.66 (d, J=15.1Hz, 1H), 4.14-4.25 (m, 1H), 4.19 (dd, J=6.1, 1.7Hz, 1H), 4.33-4.41 (m, 1H), 7.04-7.27 (m, 4H)
IR (KBr) : 3270, 2926, 2856, 2240, 1690, 1610, 1590, 1490, 1454, 1385, 1339, 1276, 1186, 1042, 1015, 958, 885, 763, 702, 622, 596 cm⁻¹

### Example 17

### 9-Deoxy-9β-bromo-3, 4, 5, 17, 18, 19, 20-heptanol-2, 6-inter-m-phenylene-16-cyclopentyl-13, 14-didehydro-PGF₁α ethyl ester (Compound 41)

(1) To an acetonitrile (1.8 ml) solution of 3, 4, 5, 17, 18, 19, 20-heptanol-2, 6-inter-m-phenylene-16-cyclopentyl-13, 14-didehydro-PGF₁α ethyl ester 11, 15-bis(tert-butyldimethylsilyl ether) (120 mg) obtained in Example 3(3) was added pyridine (30 µl), carbon tetrabromide (181 mg), and triphenylphosphine (144 mg) at room temperature followed by stirring for 3 hours. The reaction solution was poured into water and extracted with hexane-diethyl ether (1: 1). The resulting organic layer was washed with a saturated aqueous sodium chloride solution, dried over anhydrous magnesium sulfate and filtered. The filtrate was concentrated under reduced pressure and the resulting crude product was purified by silica gel column chromatography (developing solvent; hexane: ethyl acetate = 20: 1) to give 9-Deoxy-9β-bromo-3, 4, 5, 17, 18, 19, 20-heptanol-2, 6-inter-m-phenylene-16-cyclopentyl-13, 14-didehydro-PGF₁α ethyl ester 11, 15-bis(tert-butyldimethylsilyl ether) (130 mg).
   ¹H-NMR(CDCl₃, 300MHz) δ ppm; 0.08 (s, 3H), 0.10 (s, 6H), 0.13 (s, 3H), 0.89 (s, 9H), 0.90 (s, 9H), 1.00-2.47 (m, 17H), 1.26 (t, J=7.2Hz, 3H), 2.64-2.87 (m, 2H), 3.59 (s, 2H), 3.98-4.09 (m, 1H), 4.16 (q, J=7.2Hz, 2H), 4.23-4.48 (m, H), 7.06-7.30 (m, 4H)
   IR(neat) : 2952, 2930, 2857, 2232, 1740, 1609, 1472, 1387, 1365, 1337, 1253, 1151, 1083, 1035, 1006, 939, 903, 837, 777, 700, 670 cm⁻¹
(2) Following the substantially same manner as in Example 1(5) using the compound obtained in (1) above, thereby the title compound was obtained.
   ¹H-NMR (CDCl₃, 300MHz) δ ppm; 1.02-1.32 (m, 2H), 1.26 (t, J=7.2Hz, 3H), 1.40-2.51 (m, 17H), 2.67-2.90 (m, 2H), 3.59 (s, 2H), 3.97-4.08 (m, 1H), 4.15 (q, 1=7.2Hz, 2H), 4.33-4.48 (m, 2H), 7.06-7.30 (m, 4H)
   IR(neat): 3400, 2945, 2860, 2234, 1734, 1608, 1446, 1369, 1332, 1299, 1256, 1152, 1093, 1032, 769, 701 cm⁻¹

### Example 18

### 9-Deoxy-9-fluoro-3, 4, 5, 16, 17, 18, 19, 20-octanol-2, 6-inter-m-phenylene-15-cycloheptyl-13, 14-didehydro-PGF₁ ethyl ester (Compound 44)

(1) To a methylene chloride (3.6 ml) solution of 3, 4, 5, 16, 17, 18, 19, 20-octanol-2, 6-inter-m-phenylene-15-cycloheptyl-13, 14-didehydro-PGF₁α ethyl ester 11, 15-bis(tert-butyldimethylsilyl ether) (120 mg) obtained in Example 5 (3) was added diethylaminosulfur trifluoride (DAST) (118 µl) drop by drop at -78 degree C under argon stream, followed by elevating temperature to room temperature in an hour under stirring. The reaction solution was poured into a saturated aqueous sodium hydrogen carbonate solution and extracted with hexane. The resulting organic layer was washed with a saturated aqueous sodium chloride solution, dried over anhydrous magnesium sulfate and filtered. The filtrate was concentrated under reduced pressure and the resulting crude product was purified by silica gel column chromatography (developing solvent: hexane: ethyl acetate = 40: 1) to give 9-Deoxy-9-fluoro-3, 4, 5, 16, 17, 18, 19, 20-octanol-2, 6-inter-m-phenylene-15-cycloheptyl-13, 14-didehydro-PGF₁ ethyl ester 11, 15-bis(tert-butyldimethylsilyl ether) (120 mg).
   ¹H-NMR(CDCl₃, 300MHz) δ ppm; 0.07 (s, 3H) 0.09 (s, 3H), 0.10 (s, 3H), 0.11 (s, 3H), 0.89 (s, 18H), 1.15-2.53 (m, 19H), 1.25 (t, J=7.2Hz, 3H), 2.57-2.87 (m, 2H), 3.58 (s, 2H), 4.12-4.20 (m, 1H), 4.15 (q, J=7.2Hz, 2H), 4.21-4.31 (m, 1H), 4.66-4.73 (m, 0.5H), 4.84-4.91 (m, 0.5H), 7.07-7.15 (m, 3H), 7.19-7.28 (m, 1H)
   IR(neat) 2929, 2857, 2232, 1740, 1609, 1472, 1463, 1385, 1362, 1338, 1252, 1132, 1084, 1035, 1006, 939, 837, 777, 700, 671 cm⁻¹
(2) Following the substantially same manner as in Example 1(5) using the compound obtained in (1) above, thereby the title compound was obtained.
   ¹H-NMR(CDCl₃, 300MHz) δppm; 1.13-2.55 (m, 21H), 1.26 (t, J=7.2Hz, 3H), 2.65-2.90 (m, 2H), 3.59 (s, 2H), 4.15 (q, J=7.2Hz, 2H), 4.23 (dd, J=5.4, 1.5Hz, 1H), 4.27-4.39 (m, 1H), 4.66-4.73 (m, 0.5H), 4.82-4.91 (m, 0.5H), 7.06-7.29 (m, 4H)
   IR(neat) : 3400, 2924, 2856, 2236, 1734, 1608, 1590, 1446, 1369, 1333, 1300, 1255, 1152, 1095, 1033, 786, 701 cm⁻¹

### Example 19

### 9-Deoxy-9β-chloro-3, 4, 5, 16, 17, 18, 19, 20-octanol-2, 6-inter-o-phenylene-15-cyclohexyl-13, 14-didehydro-PGF₁α ethyl ester (Compound 50)

(1) In Example 1(2), following the substantially same manner as in Example 1(2) using 2-carboethoxymethylbenzylzinc(II) bromide in place of 3-carboethoxymethylbenzylzinc(II) bromide, thereby 3, 4, 5, 16, 17, 18, 19, 20-octanol-2, 6-inter-o-phenylene-15-cyclohexyl-13, 14-didehydro-PGE₁ ethyl ester 11, 15-bis(tert-butyldimethylsilyl ether) was obtained.
   ¹H-NMR(CDCL₃, 300MHz) δppm; 0.08 (s, 3H), 0.10 (s, 6H). 0.14 (s, 3H), 0.82-2.02 (m, 16H), 0.90 (s, 18H), 2.14-2.34 (m, 1H), 2.20 (dd, J=18.3, 7.1Hz, 1H), 2.65-2.89 (m, 4H), 3.68 (s, 2H), 4.10 (d, J=6.2Hz, 1H), 4.16 (q, J=7.2Hz, 2H), 4.26-4.37 (m, 1H), 7.14-7.25 (m, 4H)
   IR (neat): 2929, 2856, 2236, 1746, 1493, 1472, 1464, 1451 1369, 1336, 1252, 1154, 1100, 1032, 1006, 939, 898, 883, 838, 778, 670, 586 cm⁻¹
(2) Following the substantially same manner as in Example 1(3) using the compound obtained in (1) above, thereby 3, 4, 5, 16, 17, 18, 19, 20-octanol-2, 6-inter-o-phenylene-15-cyclohexyl-13, 14-didehydro-PGF₁α ethyl ester 11, 15-bis(tert-butyldimethylsilyl ether)
   ¹H-NMR (CDCl₃, 300MHz) δ ppm; 0.08 (s, 3H), 0.09 (s, 3H), 0.10 (s, 6H), 0.80-2.11 (m, 16H), 0.88 (s, 9H), 0.90 (s, 9H), 1.24 (t, J=7.2Hz, 3H), 2.44-2.90 (m, 4H), 3.67 (d, J=15.3Hz, 1H), 3.71 (d, J=15.3Hz, 1H), 4.04-4.30 (m, 2H), 4.08 (dd, J=6.2, 1.9Hz, 1H), 4.14 (q, J=7.2Hz, 2H), 7.12-7.27 (m, 4H).
   IR(neat) : 3468, 2929, 2855, 2232, 1736, 1472, 1463, 1451, 1388, 1368, 1336, 1252, 1155, 1103, 1066, 1006, 963, 898, 837, 777, 668 cm⁻¹ and 3, 4, 5, 16, 17, 18, 19, 20-octanol-2, 6-inter-o-phenylene-15-cyclohexyl-13, 14-didehydro-PGF₁β ethyl ester 11, 15-bis(tert-butyldimethylsilyl ether) was obtained.
   ¹H-NMR(CDCl₃, 300MHz) δppm; 0.08 (2s, 6H), 0.09 (s, 3H), 0.12 (s, 3H), 0.84-1.98 (m, 17H), 0.89 (s, 9H) (s, 9H), 1.25 (t, J=7.2Hz, 3H), 2.26-2.38 (m, 1H), 2.73-2.85(m, 2H), 3.66 (s, 2H), 4.02-29 (m, 3H), 4.15 (q, J=7.2Hz, 2H), 7.13-7.28 (m, 4H)
   IR(neat): 3436, 929, 2855, 2233, 1736, 1493, 1472, 1463, 1451, 1369, 1336, 1252, 1155, 1101, 1067, 1006, 939, 898, 837, 777, 699 cm⁻¹
(3) Following the substantially same manner as in Example 1(4) using 3, 4, 5, 16, 17, 18, 19, 20-octanol-2, 6-inter-o-phenylene-15-cyclohexyl-13, 14-didehydro-PGF₁α ethyl ester 11, 15-bis(tert-butyldimethylsilyl ether) obtained in (2) above, thereby 9-deoxy-9β-chloro-3, 4, 5, 16, 17, 18, 19, 20-octanol-2, 6-inter-o-phenylene-15-cyclohexyl-13, 14-didehydro-PGF₁α ethyl ester 11, 15-bis(tert-butyldimethylsilyl ether) was obtained.
   ¹H-NMR(CDCl₃, 300MHz) δ ppm; 0.08 (2s, 6H), 0.10 (s, 3H), 0.11 (s.3H), 0.81-1.98 (m, 13H), 0.88 (s, 9H), 0.90 (s, 9H), 1.25 (t, J=7.2Hz, 3H), 2.10-2.26 (m, 3H), 2.35-2.45 (m, 1H), 2.73-2.84 (m, 2H), 3.66 (s, 2H), 3.98-4.21 (m, 2H), 4.15 (q, J=7.2Hz, 2H), 4.24-4.34 (m, 1H), 7.14-7.25 (m, 4H)
   IR(neat): 2929, 2856, 2233, 1740, 1493, 1472, 1463, 1451, 1389, 1367, 1336, 1252, 1156, 1100, 1033, 1006, 939, 898, 837, 778, 670 cm⁻¹
(4) Following the substantially same manner as in Example 1(5) using the compound obtained in (3) above, thereby the title compound was obtained.
   ¹H-NMR(CDCl₃, 30DMHz) δ ppm; 0.96-2.55 (m, 19H), 1.25 (t,J=7.2Hz, 3H), 2.70-2.96 (m, 2H), 3.67 (s, 2H), 3.98-4.21 (m, 2H), 4.15 (q, J=7.2Hz, 2H) 4.34-4.44 (m, 1H), 7.14-7.30 (m, 4H)
   IR(neat) : 3400, 3064, 2979, 2928, 2853, 2236, 1734, 1605, 1493, 1450, 1392, 1369, 1333, 1257, 1157, 1084, 1030, 946, 893, 833, 755, 695, 633, 577 cm⁻¹

### Example 20

### 9-Deoxy-9β-chloro-3, 4, 5, 16, 17, 18, 19, 20-octanol-2, 6-inter-o-phenylene-15-cyclohexyl-13, 14-didehydro-PGF₁α (Compound 51)

Following the substantially same manner as in Example 2 using the compound obtained in Example 19(4), thereby the title compound was obtained.
¹H-NMR (CDCl₃, 300MHz) δ ppm; 0.84-1.32 (m, 6H), 1.40-2.00 (m, 10H), 2.10-2.42 (m, 4H), 2.65-2.88 (m, 2H), 3.64 (d, J=15.5Hz, 1H), 3.73 (d, J=15.5Hz, 1H) 3.95-4.07 (m, 1H), 4.13 (dd, J=6.2, 1.9Hz, 1H), 4.28-4.39 (m, 1H), 7.12-7.32 (m, 4H).
IR (neat): 3368, 3019, 2930, 2854, 2236, 1713, 1605, 1493, 1451, 1413, 1240, 1161, 1083, 1008, 946, 894, 832, 756, 668, 616 cm⁻¹

### Example 21

### 9-Deoxy-9β-chloro-3, 4, 5, 16, 17, 18, 19, 20-octanol-2, 6-inter-p-phenylene-15-cyclohexyl-13, 14-didehydro-PGF₁α ethyl ester (Compound 52)

(1) In Example 1(2), following the substantially same manner as in Example 1(2) using 4-carboethoxymethylbenzylzinc(II) bromide in place of 3-carboethoxymethylbenzylzinc(II) bromide, thereby 3, 4, 5, 16, 17, 18, 19, 20-octanol-2, 6-inter-p-phenylene-15-cyclohexyl-13, 14-didehydro-PGE, ethyl ester 11, 15-bis(tert-butyldimethylsilyl ether) was obtained. Subsequently, following the substantially same manner as in Example 1(3), 3, 4, 5, 16, 17, 18, 19, 20-octanol-2, 6-inter-p-phenylene-15-cyclohexyl-13, 14-didehydro-PGF₁α ethyl ester 11, 15-bis(tert-butyldimethylsilyl ether) was obtained.
   ¹H-NMR(CDCl₃, 300MHz) δ ppm; 0.07 (s, 3H), 0.09 (s, 6H), 0.10 (s, 3H), 0.81-2.06 (m, 16H), 0.88 (s, 9H), 0.90 (s, 9H), 1.25 (t, J=7.2Hz, 3H), 2.42-2.84 (m, 4H), 3.57 (s, 2H), 4.04-4.19 (m, 1H), 4.07 (dd, J=6.2, 1.9Hz, 1H), 4.14 (q, J=7.2Hz, 2H), 4.25-4.31 (m, 1H), 7.19 (s, 4H)
   IR(neat): 3436, 2929, 2855, 2231, 1739, 1515, 1463, 1451, 1388, 1369, 1336, 1252, 1141, 1100, 1062, 1032, 898, 837, 778, 669 cm⁻¹
(2) Following the substantially same manner as in Example 1(4) using the compound obtained in (1) above, thereby 9-deoxy-9β-chloro-3, 4, 5, 16, 17, 18, 19, 20-octanol-2, 6-inter-p-phenylene-15-cyclohexyl-13, 14-didehydro-PGF₁α ethyl ester 11, 15-bis(tert-butyldimethylsilyl ether) was obtained.
   ¹H-NMR(CDCl₃, 300MHz) δ ppm; 0.08 (s, 6H), 0.09 (s, 3H), 0.11 (s, 3H), 0.82-2.20 (m, 16H), 0.88 (s, 9H), 0.90 (s, 9H), 1.25 (t, J=7.2Hz, 3H), 2.33-2.42 (m, 1H), 2.68-2.84 (m, 2H), 3.58 (s, 2H), 3.96-4.06 (m, 1H), 4.09 (dd, J=6.2, 1.7Hz, 1H), 4.15 (q, J=7.2Hz, 2H), 4.23-4.31 (m, 1H), 7.14-7.24 (m, 4H)
   IR(neat): 2929, 2856, 2233, 1740, 1516, 1472, 1463, 1451, 1386, 1368, 1337, 1253, 1153, 1100, 1068, 1034, 1006, 939, 898, 837, 778, 669 cm⁻¹
(3) Following the substantially same manner as in Example 1(5) using the compound obtained in (2) above, thereby the title compound was obtained.
   ¹H=NMR(CDCl₃ 300MHz) δ ppm; 0.97-2.42 (m, 18H), 1.25 (t, J=7.2Hz, 3H), 2.38 (ddd, J=9.9, 6.4, 1.9Hz, 1H), 2.70-2.88 (m, 2H), 3.58 (s, 2H), 3.97-4.07 (m, 1H), 4.10-4.20 (m, 1H), 4.15 (q, J=7.2Hz, 2H), 4.33-4.44 (m, 1H), 7.14-7.24 (m, 4H)
   IR(KBr): 3376, 2926, 2855, 2234, 1738, 1517, 1453, 1410, 1369, 1338, 1284, 1222, 1155, 1110, 1014, 890, 774, 673, 608, 578 cm⁻¹

### Example 22

### 9-Deoxy-9β-chloro-3, 4, 5, 16, 17, 18, 19, 20-octanol-2, 6-inter-p-phenylene-15-cyclohexyl-13, 14-didehydro-PGF₁α (Compound 53)

Following the substantially same manner as in Example 2 using the compound obtained in Example 21(3), thereby the title compound was obtained.
¹H-NMR(CDCl₃, 300MHz) δ ppm; 0.94-2.42 (m, 20H), 2.69-2.89 (m, 2H), 3.61 (s, 2H), 3.95-4.06 (m, 1H), 4.14, (dd, J=6.1, 1.6Hz, 1H), 4.31-4.42 (m, 1H), 7.11-7.27 (m, 4H)
IR(neat): 3368, 3013, 2929, 2854, 2236, 1712, 1515 1450, 1422, 1262, 1153, 1084, 1008, 943, 894, 808, 758, 668, 610 cm⁻¹

### Example 23

### 9-Deoxy-9β-chloro-3, 4, 5, 16, 17, 18, 19, 20-octanol-2, 6-inter-o-phenylene-15-cyclopentyl-13, 14-didehydro-PGF₁α ethyl ester (Compound 34)

(1) In Example 1(2), following the substantially same manner as in Example 1(2) using 2-carboethoxymethylbenzylzinc(II) bromide in place of 3-carboethoxymethylbenzylzinc(II) bromide, and using the compound obtained in Example 7(1) in place of (3R, 4R)-2-methylene-3-[(3S)-3-(tert-butyldimethylsiloxy) -3-cyclohexylprop-1-ynyl]-4-(tert-butyldimethylsilox y)cyclopentane-1-one, thereby 3, 4, 5, 16, 17, 18, 19, 20-octanol-2, 6-inter-o-phenylene-15-cyclopentyl-13, 14-didehydro-PGE₁ ethyl ester 11, 15-bis(tert-butyldimethylsilyl ether) was obtained.
   ¹H-NMR(CDCl₃, 300MHz) δ ppm; 0.09 (s, 3H), 0.10 (s, 3H), 0.11 (s, 3H), 0.14 (s, 3H), 0.89 (s, 9H), 0.90 (s, 9H), 1.06-2.32 (m, 14H), 1.25 (t, J=7.1Hz, 3H), 2.63-2.88 (m, 2H), 3.67 (s, 2H), 4.15 (q, J=7.1Hz, 2H), 4.19 (dd, J=6.8, 1.3Hz, 1H), 4.26-4.36 (m, 1H), 7.05-7.27 (m, 4H)
   IR(neat) : 2956, 2858, 2236, 1746, 1494, 1472, 1464, 1389, 1368, 1331, 1252, 1154, 1099, 1034, 1006, 940, 887, 838, 778, 670, 576 cm⁻¹
(2) Following the substantially same manner as in Example 1(3) using the compound obtained in (1) above, thereby 3, 4, 5, 16, 17, 18, 19, 20-octanol-2, 6-inter-o-phenylene-15-cyclopentyl-13, 14-didehydro-PGF₁α ethyl ester 11, 15-bis(tert-butyldimethylsilyl ether) was obtained.
   ¹H-NMR(CDCl₃, 300MHz) δ ppm; 0.08 (s, 3H), 0.09 (s, 3H), 0.10 (s, 3H), 0.11 (s, 3H), 0.88 (s, 9H), 0.90 (s, 9H), 1.20-2.17 (m, 14H), 1.24 (t, 1=7.2Hz, 3H), 2.46-2.90 (m, 3H), 2.71 (d, J=9.0Hz, 1H), 3.65 (d, J=15.3Hz, 1H) 3.73 (d, J=15.3Hz, 1H), 4.08-4.30 (m, 3H), 4.14 (q, J=7.2Hz, 2H), 7.11-7.28 (m, 4H)
   IR(neat): 3468, 2954, 2858, 2230, 1736, 1472, 1388, 1362, 1328, 1253, 1156, 1069, 1006, 940, 838, 777, 670 cm⁻¹
(3) Following the substantially same manner as in Example 1(4) using the compound obtained in (2) above, thereby 9-deoxy-9β-chloro-3, 4, 5, 16, 17, 18, 19, 20-octanol-2, 6-inter-o-phenylene-15-cyclopentyl-13, 14-didehydro-PGF₁α ethyl ester 11, 15-bis(tert-butyldimethylsilyl ether) was obtained.
   ¹H=NMR(CDCl₃, 300MHz) δ ppm; 0.08 (s, 3H), 0.09 (s, 3H), 0.10 (s, 3H), 0.12 (s, 3H), 0.88 (s, 9H), 0.90 (s, 9H), 1.20-2.25 (m, 14H), 1.25 (t, J=7.2Hz, 3H), 2.37 (ddd, J=8.7, 4.7, 1.6Hz, 1H), 2.69-2.86 (m, 2H), 3.66 (s, 2H), 3.98-4.08 (m, 1H), 4.15 (q, J=7.2Hz, 2H), 4.19 (dd, J=6.9, 1.6Hz, 1H), 4.23-4.31 (m, 1H), 7.12-7.27 (m, 4H)
   IR(neat) : 2955, 2930, 2858, 2231, 1740, 1494, 1472, 1463, 1389, 1363, 1330, 1252, 1155, 1095, 1034, 1006, 940, 838, 778, 670 cm⁻¹
(4) Following the substantially same manner as in Example 1(5) using the compound obtained in (3) above, thereby the title compound was obtained.
   ¹H-NMR (CDCl₃, 300MHz) δ ppm; 1.20-2.53 (m, 16H), 1.25 (t, J=7.2Hz, 3H), 2.39 (ddd, J=9.9, 6.4, 1.7Hz, 1H), 2.76 (ddd, J=14.3, 11.4, 5.4Hz, 1H), 2.89 (ddd, J=14.3, 11.5, 5.5Hz, 1H), 3.67 (s, 2H) 4.15 (q, J=7.2Hz, 2H), 4.20-4.28 (m, 1H), 4.33-4.43 (m, 1H), 7.10-7.29 (m, 4H)
   IR(neat) : 3400, 3065, 2952, 2867, 2235, 1733, 1494, 1450, 1393, 1369, 1327, 1256, 1158, 1094, 1028, 940, 842, 756, 634, 574 cm⁻¹

### Example 24

### 9-Deoxy-9β-chloro-3, 4, 5, 16, 17, 18, 19, 20-octanol-2, 6-inter-o-phenylene-15-cyclopentyl-13, 14-didehydro-PGF₁α (Compound 35)

Following the substantially same manner as in Example 2 using the compound obtained in Example 23(4), thereby the title compound was obtained.
¹H-NMR (CDCl₃, 300MHz) δ ppm; 1.18-2.40 (m, 17H), 2.35 (ddd, J=10.6, 6.9, 1.8Hz, 1H), 2.66-2.89 (m, 2H), 3.64 (d, J=16.2Hz, 1H), 3.73 (d, J=16.2Hz, 1H), 3.97-4.08 (m, 1H), 4.19-4.38 (m, 2H), 7.13-7.30 (m, 4H)
IR(neat): 3368, 3020, 2952, 2867, 2236, 1712, 1493, 1451, 1413, 1323, 1240, 1167, 1084, 1025, 940, 756, 669, 616 cm⁻¹

### Example 25

### 9-Deoxy-9β-chloro-3, 4, 5, 16, 17, 18, 19, 20-octanol-2, 6-inter-o-phenylene-15-cycloheptyl-13, 14-didehydro-PGF₁α ethyl ester (Compound 63)

(1) In Example 1(2), following the substantially same manner as in Example 1(2) using 2-carboethoxymethylbenzylzinc(II) bromide in place of 3-carboethoxymethylbenzylzinc(II) bromide, and using the compound obtained in Example 5(1) in place of (3R, 4R)-2-methylene-3-[(3S)-3-(tert-butyldimethylsiloxy) -3-cyclohexylprop-1-ynyl]-4-(tert-butyldimethylsilox y)cyclopentane-1-one, thereby 3, 4, 5, 16, 17, 18, 19, 20-octanol-2, 6-inter-o-phenylene-15-cycloheptyl-13, 14-didehydro-PGE₁ ethyl ester 11, 15-bis(tert-butyldimethylsilyl ether) was obtained.
   ¹H-NMR(CDCl₃, 300MHz) δ ppm; 0.08 (s, 3H), 0.10 (s, 6H), 0.14 (s, 3H), 0.89 (s, 9H), 0.90 (s, 9H), 1.08-2.08 (m, 15H), 1.25 (t, J=7.2Hz, 3H), 2.13-2.34 (m, 1H), 2.20 (dd, J=18.2, 6.8Hz, 1H), 2.63-2.88 (m, 4H), 3.67 (s, 2H), 3.98-4.20 (m, 1H), 4.15 (q, J=7.2Hz, 2H), 4.25-4.36 (m, 1H), 7.00.-7.27 (m, 4H)
   IR(neat): 2929, 2857, 2236, 1745, 1493, 1472, 1463, 1368, 1336, 1252, 1154, 1122, 1085, 1033, 1006, 939, 883, 838, 778, 670 cm⁻¹
(2) Following the substantially same manner as in Example 1(3) using the compound obtained in (1) above, thereby 3, 4, 5, 16, 17, 18, 19, 20-octanol-2, 6-inter-o-phenylene-15-cycloheptyl-13, 14-didehydro-PGF₁α ethyl ester 11, 15-bis(tert-butyldimethylsilyl ether) was obtained.
   ¹H-NMR(CDCl₃, 300MHz) δ ppm; 0.08 (s, 3H) 0.09 (s, 3H), 0.10 (s, 3H), 0.11 (s, 3H), 0.88 (s, 9H), 0.89 (s, 9H), 1.19-2.11 (m, 18H), 1.24 (t, J=7.1Hz, 3H), 2.42-2.91 (m, 3H), 2.70 (d, J=8.9Hz, 1H), 3.66 (d, J=15.3Hz, 1H), 3.73 (d, J=15.3Hz, 1H), 4,07-4.30 (m, 3H), 4.14 (q, J=7.1Hz, 2H), 7.11-7.28 (m, 4H)
   IR(neat): 3468, 2929, 2858, 2227, 1737, 1463, 1386, 1362, 1336, 1252, 1084, 1034, 1006, 837, 776, 666, 502 cm⁻¹
(3) Following the substantially same manner as in Example 1(4) using the compound obtained in (2) above, thereby 9-deoxy-9β-chloro-3, 4, 5, 16, 17, 18, 19, 20-octanol-2, 6-inter-o-phenylene-15-cycloheptyl-13, 14-didehydro-PGF₁α ethyl ester 11, 15-bis(tert-butyldimethylsilyl ether) was obtained.
   ¹H-NMR (CDCl₃, 300MHz) δppm; 0.08 (s, 3H), 0.09 (s, 3H), 0.10 (s, 3H), 0.11 (s, 3H), 0.88 (s, 9H), 0.89 (s, 9H), 1.18-1.99 (m, 15H), 1.25 (t, J=7.2Hz, 3H), 2.10-2.26 (m, 2H), 2.39 (ddd,J=8.9, 4.8, 1.6Hz, 1H), 2.69-2.86.(m, 2H), 3.66 (s, 2H), 3.98-4.09 (m, 1H), 4.15 (q, J=7.2Hz, 2H), 4.17 (dd, J=5.4, 1.6Hz, 1H), 4.24-4.32 (m, 1H), 7.12-7.27 (m, 4H)
   IR(neat) : 2929, 285.7, 2230, 1740, 1494, 1463, 1388, 1363, 1336, 1252, 1155, 1085, 1034, 1006, 940, 837, 777, 670 cm⁻¹.
(4) Following the substantially same manner as in Example 1(5) using the compound obtained in (3) above, thereby the title compound was obtained.
   ¹H-NMR(CDCl₃, 300MHz) δ ppm; 1.16-2.47 (m, 20H), 1.25 (t, J=7.2Hz, 3H), 2.38 (d, J=5.1Hz, 1H), 2.77 (ddd, J=14.3, 11.3, 5.4Hz, 1H), 2.89 (ddd, J=14.3, 11.7, 5.5Hz, 1H), 3.67 (s, 2H), 4.15 (q, J=7.2Hz, 2H), 3.98-4.27 (m, 2H), 4.33-4.43 (m, 1H), 7.13-7.29 (m, 4H)
   IR(neat) : 3400, 2924, 2856, 2233, 1734, 1493, 1446, 1369, 1332, 1256, 1157, 1094, 1030, 755 cm⁻¹

### Example 26

### 9-Deoxy-9β-chloro-3, 4, 5, 16, 17, 18, 19, 20-octanol-2, 6-inter-o-phenylene-15-cycloheptyl-13, 14-didehydro-PGF₁α (Compound 64)

Following the substantially same manner as in Example 2, using the compound obtained in Example 25(4), thereby the title compound was obtained.
¹H-NMR (CDCl₃, 300MHz) δ ppm; 1.20-2.44 (m, 22H), 2.35 (ddd, J=10.9, 6.8, 2.3Hz, 1H), 2.70-2.86 (m, 2H), 3.59-3.80 (m, 2H), 3.98-4.15 (m, 1H), 4.18-4.39 (m, 2H), 7.15-7.29 (m, 4H)
IR (neat): 3368, 3019, 2924, 2857, 2235, 1712, 1493, 1456, 1413, 1239, 1170, 1047, 1014, 942, 756, 668, 616 cm⁻¹

### Example 27

### 9-Deoxy-9β-chloro-3, 4, 16, 17, 18, 19, 20-heptanol-2, 5-inter-m-phenylene-15-cyclohexyl-13, 14-didehydro-PGF₁α methyl ester (Compound 75)

(1) In Example 1(2), following the substantially same manner as in Example 1(2) using 3-carbomethoxymethylphenethylzinc(II) iodide in place of 3-carboethoxymethylbenzylzinc(II) bromide, thereby 3, 4, 16, 17, 18, 19, 20-heptanol-2, 5-inter-m-phenylene-15-cyclohexyl-13, 14-didehydro-PGE₁ methyl ester 11, 15-bis(tert-butyldimethylsilyl ether) was obtained.
   ¹H-NMR (CDCl₃, 300MHz) δ ppm; 0.07 (s, 3H), 0.08 (s, 3H), 0.09 (s, 3H), 0.11 (s, 3H), 0.72-1.92 (m, 15H), 0.88 (s, 9H), 0.90 (s, 9H), 2.08-2.29 (m, 1H), 2.16 (dd, J=18.1, 1.7Hz, 1H), 2.50-2.74 (m, 2H), 3.59 (s, 2H), 3.69 (s, 3H), 4.08 (dd, J=6.2, 1.6Hz, 1H), 4.21-4.34 (m, 1H), 7.03-7.28 (m, 4H)
   IR(neat) : 2930, 2856, 2236, 1746, 1609, 1472, 1463, 1450, 1386, 1339, 1255, 1100, 1007, 940, 898, 838, 778, 700, 670 cm⁻¹
(2) Following the substantially same manner as in Example 1(3) using the compound obtained in (1) above, thereby 3, 4, 16, 17, 18, 19, 20-heptanol-2, 5-inter-m-phenylene-15-cyclohexyl-13, 14-didehydro-PGF₁α methyl ester 11, 15-bis(tert-butyldimethylsilyl ether) was obtained.
   ¹H-NMR (CDCl₃, 300MHz) δppm; 0.07 (s, 3H), 0.08 (s, 3H), 0.09 (s, 3H), 0.10 (s, 3H), 0.74-2.06 (m, 18H), 0.88 (s, 9H), 0.89 (s, 9H), 2.38-2.59 (m, 2H), 2.64 (t, J=7.2Hz, 2H), 3.60 (s, 2H), 3.69 (s, 3H), 4.06 (dd, J=6.4, 1.9Hz, 1H), 4.00-4.16 (m, 1H), 4.24-4.32 (m, 1H), 7.05-7.27 (m, 4H)
   IR (neat) : 3468, 3232, 2929, 2856, 2231, 1743, 1608, 1463, 1450, 1387, 1361, 1337, 1256, 1104, 1067, 1006, 898, 837, 777, 668 cm⁻¹
(3) Following the substantially same manner as in Example 1(4) using the compound obtained in (2) above, thereby 9-deoxy-9β-chloro-3, 4, 16, 17, 18, 19, 20-heptanol-2, 5-inter-m-phenylene-15-cyclohexyl-13, 14-didehydro-PGF₁α methyl ester 11, 15-bis(tert-butyldimethylsilyl ether) was obtained.
   ¹H-NMR(CDCl₃, 300MHz) δppm; 0.06 (s, 3H), 0.07 (s, 3H), 0.08 (s, 3H), 0.10 (s, 3H), 0.74-1.89 (m, 15H), 0.87 (s, 9H), 0.90 (s, 9H), 2.03-2.22 (m, 3H), 2.29 (ddd, J=8.9, 5.0, 1.6Hz, 1H), 2.63 (t, J=7.5Hz, 2H), 3.60 (s, 2H), 3.69 (s, 3H), 3.88-4.00 (m, 1H), 4.07 (dd, J=6.3, 1.6Hz, 1H), 4.18-4.30 (m, 1H), 7.02-7.28 (m, 4H)
   IR(neat): 2930, 2855, 2232, 1746, 1609, 1472, 1463, 1450, 1362, 1338, 1256, 1152, 1100, 1007, 962, 940, 898, 837, 778, 700, 669 cm⁻¹
(4) Following the substantially same manner as in Example 1(5) using the compound obtained in (3) above, thereby the title compound was obtained.
   ¹H-NMR(CDCl₃, 300MHz) δ ppm; 0.88-3.00 (m, 21H), 2.64 (t, J=7.5Hz, 2H), 3.60 (s, 2H), 3.69 (s, 3H), 3.88-4.01 (m, 1H), 4.13 (dd, J=6.0, 1.8Hz, 1H), 4.28-4.42 (m, 1H), 7.02-7.28 (m, 4H)
   IR(neat) : 3400, 2928, 2853, 2235, 1740, 1608, 1590, 1489, 1449, 1261, 1203, 1153, 1084, 1013, 951, 893, 771, 700 cm⁻¹

### Example 28

### 9-Deoxy-9β-chloro-3, 4, 16, 17, 18, 19, 20-heptanol-2, 5-inter-m-phenylene-15-cyclohexyl-13, 14-didehydro-PGF₁α (Compound 76)

Following the substantially same manner as in Example 2 using the compound obtained in Example 27(4), thereby the title compound was obtained.
¹H-NMR(CDCl₃, 300MHz) δ ppm; 0.84-2.32 (m, 22H), 2.51-2.74 (m, 2H), 3.62 (s, 2H), 3.88-3.98 (m, 1H), 4.07-4.17 (m, 1H), 4.29-4.40 (m, 1H), 7.04-7.28 (m, 4H)
IR (neat) : 3368, 2930, 2854, 2236, 1713, 1609, 1590, 1489, 1450, 1408, 1259, 1155, 1084, 1008, 948, 894, 758, 704, 668, 613 cm⁻¹

### Example 29

### 9-Deoxy-9β-chloro-3, 4, 16, 17, 18, 19, 20-heptanol-2, 5-inter-o-phenylene-15-cyclohexyl-13, 14-didehydro-PGF₁α methyl ester (Compound 77)

(1) In Example 1(2), following the substantially same manner as in Example 1(2) using 2-carbomethoxymethylphenethylzinc(II) iodide in place of 3-carboethoxymethylbenzylzinc(II) bromide, thereby 3, 4, 16, 17, 18, 19, 20-heptanol-2, 5-inter-o-phenylene-15-cyclohexyl-13, 14-didehydro-PGE, methyl ester 11, 15-bis(tert-butyldimethylsilyl ether) was obtained.
   ¹H-NMR (CDCl₃, 300MHz) δ ppm; 0.07 (s, 3H), 0.08 (s, 3H), 0.90 (s, 3H), 0.11 (s, 3H), 0.82-1.92 (m, 15H), 0.88 (s, 9H), 0.89 (s, 9H), 2.10-2.30 (m, 1H), 2.16 (dd, J=18.3, 6.9Hz, 1H), 2.57-2.75 (m, 2H), 3.63 (t, J=7.3Hz, 2H), 3.65 (s, 2H), 3.69 (s, 3H), 4.08 (dd, J=6.3, 1.5Hz, 1H), 4.24-4.34 (m, 1H), 7.11-7.29 (m, 4H)
   IR(neat) : 3232, 2929, 2856, 2344, 1746, 1630, 1493, 1463, 1385, 1337, 1252, 1156, 1101, 1036, 1006, 940, 898, 838, 778, 670 cm⁻¹
(2) Following the substantially same manner as in Example 1(3) using the compound obtained in (1) above, thereby 3, 4, 16, 17, 18, 19, 20-heptanol-2, 5-inter-o-phenylene-15-cyclohexyl-13, 14-didehydro-PGF₁α methyl ester 11, 15-bis(tert-butyldimethylsilyl ether) was obtained.
   ¹H-NMR(CDCl₃, 300MHz) δppm; 0.07 (s, 3H), 0.08 (s, 3H), 0.90 (s, 3H), 0.10 (s, 3H), 0.80-1.89 (m, 17H), 0.88 (s, 9H), 0.89 (s, 9H), 1.95-2.06 (m, 1H), 2.42-2.73 (m, 4H), 3.67 (s, 2H), 3.68 (s, 3H), 4.03-4.17 (m, 1H), 4.07 (dd, J=6.2, 1.9Hz, 1H), 4.24-4.30 (m, 1H), 7.11-7.24 (m, 4H)
   IR(neat) : 3790, 3228, 2929, 2855, 2366, 1740, 1630, 1462, 1386, 1337, 1294, 1253, 1103, 1063, 1036, 898, 837, 777, 668, 629, 596 cm⁻¹
(3) Following the substantially same manner as in Example 1(4) using the compound obtained in (2) above, thereby 9-deoxy-9β-chloro-3, 4, 16, 17, 18, 19, 20-heptanol-2, 5-inter-o-phenylene-15-cyclohexyl-13, 14-didehydro-PGF₁α methyl ester 11, 15-bis(tert-butyldimethylsilyl ether) was obtained.
   ¹H-NMR(CDCl₃, 300MHz) δ ppm; 0.06 (s, 6H), 0.08 (s, 3H), 0.11 (s, 3H), 0.80-1.88 (m, 15H), 0.87 (s, 9H), 0.89 (s, 9H), 2.03-2.22 (m, 3H), 2.29 (ddd, J=8.8, 5.1, 1.7Hz, 1H), 2.52-2.70 (m, 2H), 3.66 (s, 2H), 3.69 (s, 3H, 3.89-4.01 (m, 1H), 4.07 dd, J=6.3, 1.7Hz, 1H), 4.21-4.29 (m, 1H), 7.11-724 (m, 4H)
   IR(neat): 2930, 2855, 2232, 1743, 1494, 1472, 1463, 1451, 1389, 1362, 1338, 1253, 1157, 1101, 1006, 961, 940, 898, 837, 778, 670 cm⁻¹
(4) Following the substantially same manner as in Example 1(5) using the compound obtained in (3) above, thereby the title compound was obtained.
   ¹H-NMR (CDCl₃, 300MHz) δ ppm; 0.84-2.34 (m, 21H), 2.66 (t, J=7.5Hz, 2H), 3.68 (s, 2H), 3.70 (s, 3H), 3.90-4.00 (m, 1H), 4.11 (dd, J=6.0, 1.8Hz, 1H), 4.31-4.42 (m, 1H), 7.12-7.28 (m, 4H)
   IR(neat): 3400, 3021, 12929, 2853, 2236, 1735, 1493, 1450, 1336, 1260, 1205, 1159 1084, 1012, 950, 894, 834, 752, 693, 578 cm⁻¹

### Example 30

### 9-Deoxy-9β-chloro-3, 4, 16, 17, 18, 19, 20-heptanol-2, 5-inter-o-phenylene-15-cyclohexyl-13, 14-didehydro-PGF₁α (Compound 78)

Following the substantially same manner as in Example 2 using the compound obtained in Example 29(4), thereby the title compound was obtained.
¹H-NMR(CDCl₃, 300MHz) δ ppm; 0.82-1.88 (m, 15H), 2.03-2.33 (m, 3H), 2.28 (ddd, J=10.1 6.6, 1.5Hz, 1H), 2.56-2.74 (m, 2H), 3.67 (s, 2H), 3.84-4.60 (m, 5H), 4.07 (dd, J=6.3, 1.5Hz, 1H), 7.13-7.27 (m, 4H)
IR(KBr): 3403, 2925, 2856, 2234, 1698, 1493, 1450, 1415, 1341, 1280, 1243, 1210, 1176, 1114, 1086, 1004, 952, 912, 893, 798, 750, 711, 680, 581 cm⁻¹

### Example 31

### 9-Deoxy-9β-chloro-3, 4, 5, 6, 16, 17, 18, 19, 20-nonanol-2, 7-inter-m-phenylene-15-cyclohexyl-13, 14-didehydro-PGF₁α methyl ester (Compound 25)

(1) In Example 1(2), following the substantially same manner as in Example 1(2) using 3-carbomethoxymethylphenylzinc(II) iodide in place of 3-carboethoxymethylbenzylzinc(II) bromide, thereby 3, 4, 5, 6, 16, 17, 18, 19, 20-nonanol-2, 7-inter-m-phenylene-15-cyclohexyl-13, 14-didehydro-PGE₁ methyl ester 11, 15-bis(tert-butyldimethylsilyl ether) was obtained.
   ¹H-NMR(CDCl₃, 300MHz) δ ppm; 0.04 (s, 3H), 0.06 (s, 3H), 0.09 (s, 3H), 0.11 (s, 3H), 0.69-1.89 (m, 15H), 0.87 (s, 9H), 0.88 (s, 9H), 2.16 (dd, J=18.0, 6.4Hz, 1H), 2.49-2.60 (m, 1H), 2.63-2.79 (m, 2H), 2.96 (dd, J=14.9, 7.3Hz, 1H), 3.16 (dd, J=14.9, 5.4Hz, 1H), 3.67 (s, 3H), 3.71 (d, J=17.0Hz, 1H), 3.78 (d, J=17.0Hz, 1H), 4.00 (dd, J=6.3, 1.3Hz, 1H), 4.23-4.37 (m, 1H), 7.05-7.42 (m, 4H)
   IR (neat) : 2930, 2856, 2236, 1746, 1609, 1494, 1472, 1463, 1452, 1436, 1362, 1339, 1252, 1157, 1006, 939, 898, 879, 838, 779, 670, 578 cm⁻¹
(2) Following the substantially same manner as in Example 1(3) using the compound obtained in (1) above, thereby 3, 4, 5, 6, 16, 17, 18, 19, 20-nonanol-2, 7 -inter-m-phenylene-15-cyclohexyl-13, 14-didehydro-PGF₁α methyl ester 11, 15-bis(tert-butyldimethylsilyl ether) was obtained.
   ¹H-NMR(CDCl₃, 300MHz) δ ppm; 0.09 (s, 3H) 0.10 (s, 3H), 0.11 (s, 3H), 0.13 (s, 3H), 0.76-2.08 (m, 14H), 0.90 (s, 9H), 0.91 (s, 9H), 2.54-2.77 (m, 2H), 2.92 (dd, J=14.1, 4.8Hz, 1H), 3.01 (dd, J=14.1, 10.5Hz, 1H), 3.69 (s, 3H), 3.84-3.94 (m, 1H), 3.74 (d, J=15.7Hz, 1H), 3.84 (d, J=15.7Hz, 1H), 4.09 (dd, J=6.2, 1.6Hz, 1H), 4.22-4.34 (m, 1H), 7.04-7.36 (m, 4H)
   IR(neat): 3523, 2929, 2855, 2231, 1741, 1495, 1472, 1463, 1451, 1388, 1361, 1338, 1255, 1160, 1103, 1006, 938, 898, 837, 778, 668 cm⁻¹
(3) Following the substantially same manner as in Example 1(4) using the compound obtained in (2) above, thereby 9-deoxy-9β-chloro-3, 4, 5, 6, 16, 17, 18, 19, 20-nonanol-2, 7-inter-m-phenylene-15-cyclohexyl-13, 14-didehydro-PGF₁α methyl ester 11, 15-bis(tert-butyldimethylsilyl ether) was obtained.
   ¹H=NMR (CDCl₃, 300MHz) δppm; 0.05 (s, 3H), 0.06 (s, 3H) 0.07 (s, 3H), 0.08 (s, 3H), 0.64-1.84 (m, 11H), 0.86 (s, 9H) 0.89 (s, 9H), 2.00-2.46 (m, 1H), 2.90 (dd, J=14.6, 6.5Hz, 1H), 3.04 (dd, J=14.6, 5.3Hz, 1H), 3.68 (s, 3H) 3.72 (d, J=15.5Hz, 1H), 3.77 (d, J=15.5Hz, 1H), 3.91-4.04 (m, 2H), 4.21-4.29 (m, 1H), 7.05-7.30 (m, 4H)
   IR(neat) : 2930, 2855, 2235, 1745, 1495, 1472, 1463, 1452, 1389, 1362, 1338, 1253, 1158, 1100, 1006, 963, 939, 898, 837, 778, 669 cm⁻¹
(4) Following the substantially same manner as in Example 1 (5) using the compound obtained in (3) above, thereby the title compound was obtained.
   ¹H-NMR (CDCl₃, 300MHz) δppm; 0.84-2.48 (m, 17H), 2.86 (dd, J=14.3, 7.2Hz, 1H), 3.07 (dd, J=14.3, 5.2Hz, 1H), 3.70 (s, 3H), 3.73 (d, J=15.9Hz, 1H), 3.81 (d, J=15.9Hz, 1H), 3.90-4.07 (m, 2H), 4.26-4.38 (m, 1H), 7.15-7.36 (m, 4H)
   IR(neat) : 3400, 2929, 2853, 2236, 1735, 1494, 1450, 1336, 1262, 1160, 1083, 1010, 957, 893, 870, 834, 758, 694, 577 cm⁻¹

### Example 32

### 9-Deoxy-9β-chloro-3, 4, 5, 6, 16, 17, 18, 19, 20-nonanol-2, 7-inter-m-phenylene-15-cyclohexyl-13, 14-didehydro-PGF₁α (Compound 26)

Following the substantially same manner as in Example 2 using the compound obtained in Example 31(4), thereby the title compound was obtained.
¹H-NMR (CDCl₃, 300MHz) δ ppm; 0.83-1.85 (m, 14H), 2.12-2.51 (m, 3H), 2.78-3.58 (m, 2H), 2.86 (dd, J=14.3, 7.4Hz, 1H), 3.08 (dd, J=14.3, 5.3Hz, 1H), 3.75 (d, J=16.2Hz, 1H), 3.82 (d, J=16.2Hz, 1H), 3.94-4.07 (m, 1H) 3.97 (dd, J=6.1, 1.7Hz, 1H), 4.25-4.37 (m, 1H), 7.13-7.31 (m, 4H)
IR(neat): 3339, 2929, 2856, 2240, 1698, 1526, 1453, 1331, 1144, 1114, 1011, 892, 756, 695, 622, 572 cm⁻¹

### Example 33

### 9-Deoxy-9α-chloro-3, 4, 5, 16, 17, 18, 19, 20-octanol-2, 6-inter-o-phenylene-15-cyclohexyl-13, 14-didehydro-PGF₁β ethyl ester (Compound 91)

(1) Following the substantially same manner as in Example 1(4) using 3, 4, 5, 16, 17, 18, 19, 20-octanol-2, 6-inter-o-phenylene-15-cyclohexyl-13, 14-didehydro-PGF₁β ethyl ester 11, 15-bis(tert-butyldimethylsilyl ether) obtained in Example 19(2), thereby 9-deoxy-9α-chloro-3, 4, 5, 16, 17, 18, 19, 20-octanol-2, 6-inter-o-phenylene-15-cyclohexyl-13, 14-didehydro-PGF₁β ethyl ester 11, 15-bis(tert-butyldimethylsilyl ether) was obtained.
   ¹H-NMR(CDCl₃, 300MHz) δ ppm: 0.06 (s, 3H), 0.07 (s, 3H), 0.09 (s, 6H), 0.81-2.09 (m, 15H), 0.89 (s, 18H), 1.25 (t, J=7.2Hz, 3H), 2.47-2.89 (m, 4H), 3.64 (d, J=15.6Hz, 1H), 3.73 (d, J=15.6Hz, 1H), 4.08 (dd, 1=6.1, 1.6Hz, 1H), 4.15 (q, J=7.2Hz, 2H), 4.20 (ddd, J=8.3, 6.9, 3.7Hz, 1H), 4.36-4.43 (m, 1H), 7.09-7.30 (m, 4H)
   IR(neat): 2929, 2856, 2234, 1738, 1494, 1472, 1463, 1452, 1368, 1336, 1252, 1157, 1102, 1032, 1006, 939, 887, 837, 777, 668, 629 cm⁻¹
(2) Following the substantially same manner as in Example 1(5) using the compound obtained in (1) above, thereby the title compound was obtained.
   ¹H-NMR (CDCl₃, 300MHz) δ ppm; 0.94-1.34 (m, 6H), 1.25 (t, J=7.2Hz, 3H), 1.44-2.34 (m, 11H), 2.59-2.87 (m, 4H), 3.64 (d, J=15.4Hz, 1H), 3.72 (d, J=15.4Hz, 1H), 4.10-4.20 (m, 1H), 4.15 (q, J=7.2Hz, 2H), 4.23-4.33 (m, 1H), 4.44-4.50 (m, 1H), 7.14-7.28 (m, 4H)
   IR (neat) : 3385, 3062, 2927, 2853, 2236, 1732, 1493, 1450, 1369, 1333, 1258, 1158, 1095, 1030, 893, 835, 756, 692, 630, 576 cm⁻¹

### Example 34

### 9-Deoxy-9α-chloro-3, 4, 5, 16, 17, 18, 19, 20-octanol-2, 6-inter-o-phenylene-15-cyclohexyl-13, 14-didehydro-PGF₁β (Compound 92)

Following the substantially same manner as in Example 2 using the compound obtained in Example 33(2), thereby the title compound was obtained.
¹H-NMR (CDCl₃, 300MHz) δ ppm; 0.78-2.30 (m, 5H), 2.52-2.88 (m, 4H), 3.59 (d, J=15.9Hz, 1H), 3.74 (d, J=15.9Hz, 1H), 4.07-4.28 (m, 2H), 4.45-4.53 (m, 1H), 4.65-6.20 (br s, 3H), 7.07-7.40 (m, 4H)
IR(neat): 3383, 3020, 2928, 2854, 2237, 1714, 1493, 1451, 1415, 1260, 1163, 1082, 1006, 893, 834, 756, 668, 616 cm⁻¹

### Example 35

### 9-Deoxy-9β-chloro-3, 4, 5, 16, 17, 18, 19, 20-octanol-2, 6-inter-m-(3-methylphenylene)-15-cyclohexyl-13, 14-didehydro-PGF₁α ethyl ester (Compound 42)

(1) In Example 1(2), following the substantially same manner as in Example 1(2) using 3-carboethoxymethyl-5-methylbenzylzinc(II) bromide in place of 3-carboethoxymethylbenzylzinc(II) bromide, thereby 3, 4, 5, 16, 17, 18, 19, 20-octanol-2, 6-inter-m-(3-methylphenylene)-15-cyclohexyl-13, 14-didehydro-PGE₁ ethyl ester 11, 15-bis(tert-butyldimethylsilyl ether) was obtained.
   ¹H-NMR(CDCl₃, 300MHz) δ ppm: 0.08 (s, 3H), 0.09 (s, 3H), 0.10 (s, 3H), 0.13 (s, 3H), 0.80-2.39 (m, 14H), 0.90 (s, 18H), 1.25 (t, J=7.2Hz, 3H), 2.30 (s, 3H), 2.59-2.81 (m, 4H), 3.53 (s, 2H), 4.09 (dd, J=6.4, 1.7Hz, 1H), 4.14 (q, J=7.2Hz, 2H), 4.26-4.35 (m, 1H), 6.88-6.97 (m, 3H)
   IR(neat) : 2930, 2857, 2237, 1746, 1606, 1472, 1463, 1368, 1338, 1252, 1110, 1036, 1006, 939, 898, 882, 839, 778, 670, 578 cm⁻¹
(2) Following the substantially same manner as in Example 1(3) using the compound obtained in (1) above, thereby 3, 4, 5, 16, 17, 18, 19, 20-octanol-2, 6-inter-m-(3-methylphenylene)-15-cyclohexyl-13, 14-didehydro-PGF₁α ethyl ester 11, 15-bis(tert-butyldimethylsilyl ether) was obtained.
   ¹H-NMR(CDCl₃, 300MHz) δ ppm; 0.08 (s, 3H), 0.09 (s, 6H), 0.11 (s, 3H), 0.76-2.06 (m, 16H), 0.88 (s, 9H), 0.90 (s, 9H), 1.25 (t, J=7.2Hz, 3H), 2.31 (s, 3H), 2.44-2.80 (m, 4H), 3.54 (s, 2H), 4.00-4.20 (m, 1H), 4.07 (dd, J=6.3, 1.9Hz, 1H), 4.15 (q, 1=7.2Hz, 2H), 4.25-4.32 (m, 1H), 6.83-6.97 (m, 3H)
   IR(neat): 3529, 2929, 2856, 2232, 1738, 1605, 1472, 1463, 1388, 1368, 1251, 1138, 1101, 1066, 1006, 963, 939, 898, 837, 777, 668 cm⁻¹
(3) Following the substantially same manner as in Example 1(4) using the compound obtained in (2) above, thereby 9-deoxy-9β-chloro-3, 4, 5, 16, 17, 18, 19, 20-octanol-2, 6-inter-m-(3-methylphenylene)-15-cyclohexyl-13, 14-didehydro-PGF₁α ethyl ester 11, 15-bis(tert-butyldimethylsilyl ether) was obtained.
   ¹H-NMR(CDCl₃, 300MHz) δ ppm; 0.08 (s, 6H), 0.10 (s, 3H), 0.11 (s, 3H), 0.80-2.22 (m, 16H), 0.88 (s, 9H), 0.90 (s, 9H), 1.26 (t, J=7.2Hz, 3H), 2.31 (s, 3H), 2.37 (ddd, J=8.9, 4.7, 1.8Hz, 1H), 2.62-2.80 (m, 2H), 3.54 (s, 2H), 3.95-4.09 (m, 1H), 4.09 (dd, J=6.2, 1.8Hz, 1H), 4.15 (q, J=7.2Hz, 2X), 4.23-4.32 (m, 1H), 6.86-6.99 (m, 3H)
   IR(neat) : 2929, 2856, 2234, 1740, 1606, 1472, 1463, 1366, 1338, 1252, 1152, 1099, 1069, 1037, 1006, 939, 898, 837, 778, 669 cm⁻¹
(4) Following the substantially same manner as in Example 1(5) using the compound obtained in (3) above, thereby the title compound was obtained.
   ¹H-NMR(CDCl₃, 300MHz) δ ppm; 0.82-3.35 (m, 21H), 1.26 (t, J=7.2Hz, 3H), 2.31 (s, 3H), 3.55 (s, 2H), 3.94-4.05 (m, 1H), 4.07-4.20 (m, 1H), 4.15 (q, J=7.2Hz, 2H), 4.23-4.44 (m, 1H, 6.85-7.01 (m, 3H)
   IR (neat) : 3384/2926, 2854, 2236, 1732, 1605, 1451, 1369, 1296, 1255, 1152, 1085, 1033, 893, 858, 774, 703, 578 cm⁻¹

### Example 36

### 9-Deoxy-9β-chloro-3, 4, 5, 16, 17, 18, 19, 20-octanol-2, 6-inter-m-(3-methylphenylene)-15-cyclohexyl-13, 14-didehydro-PGF₁α (Compound 43)

Following the substantially same manner as in Example 2 using the compound obtained in Example 35(4), thereby the title compound was obtained.
¹H-NMR(CDCl₃ 300MHz) δ ppm; 0.94-2.40 (m, 17H), 2.37(s, 3H), 2.67-2.84 (m, 2H), 3.58 (s, 2H), 3.94-4.90 (m, 5H), 4.17 (dd, J=6.1, 1.7Hz, 1H), 6.89-6.99 (m, 3H)
IR (neat): 3333, 2926, 2852, 2237, 1708, 1606, 1456, 1412, 1372, 1290, 1223, 1145, 1114, 1020, 894, 850, 773, 711, 692, 646 cm⁻¹

### Example 37

### 9-Deoxy-9β-chloro-3, 4, 5, 16, 17, 18, 19, 20-octanol-2, 6-inter-m-phenylene-15-cyclohexyl-13, 14-didehydro-PGF₁α methyl ester (Compound 48)

To a methanol (7.9 ml) solution of the compound (330 mg) obtained in Example 2, added a catalytic quantity of concentrated sulfuric acid, followed by stirring at room temperature for 5 days. The reaction solution was added to a saturated aqueous sodium hydrogen carbonate solution and extracted with n-hexane: ethyl acetate (1:1). The organic layer was washed with water and a saturated aqueous sodium chloride solution, dried over anhydrous magnesium sulfate and filtrated. The filtrate was concentrated under reduced pressure and the resulting residue was purified by silica gel column chromatography (developing solvent: n-hexane: ethyl acetate = 2: 1) to give the title compound (310 mg).
¹H-NMR (CDCl₃, 300MHz) δ ppm; 0.95=1.36 (m, 5H), 1.46-2.07(m, 9H), 213-2.42 (m, 4H), 2.37 (ddd, J=9.8, 6.4, 1.9Hz, 1H), 2.70-2.90 (m, 2H), 3.61 (s, 2H), 3.70(s, 3H), 3.95-4.06 (m, 1H), 4.17 (dt, J=1.9, 5.4Hz, 1H), 4.38 (ddd, J=12.9, 6.4, 3.7Hz, 1H), 7.06-7.18 (m, 3H), 7.25 (t, J=7.5Hz, 1H)
IR (neat) : 3385, 2927, 2853, 2236, 1738, 1608, 1590, 1489, 1449, 1336, 1263, 1203, 1152, 1084, 1013, 893, 773, 716, 700 cm⁻¹

### Example 38

### 9-Deoxy-9β-chloro-3, 4, 5, 16, 17, 18, 19, 20-octanol-2, 6-inter-m-phenylene-15-cyclohexyl-13, 14-didehydro-PGF₁α isopropyl ester (Compound 45)

In example 37, following the substantially same manner as in Example 37 using isopropanol in place of methanol, thereby the title compound was obtained.
¹H-NMR(CDCl₃, 300MHz) δ ppm; 0.97-1.34 (m, 5H), 1.23 (d, J=6.2Hz, 6H), 1.42-2.06 (m, 9H), 2.11-2.42 (m, 4H), 2.(37 (ddd, J=9.8, 6.4, 1.8Hz, 1H), 2.70-2.89 (m, 2H), 3.56 (s, 2H), 3.95-4.06 (m, 1H), 4.12-4.21 (m, 1H), 4.32-43 (m, 1H), 5.01 (sept, J=6.2Hz, 1H), 7.07-7.29 (m, (4H)
IR(neat): 3389, 2981, 2928, 2854, 2236, 1731, 1608, 1590, 1490, 1451, 1374, 1263, 1146, 1107, 1012, 972, 894, 830, 774, 700 cm⁻¹

### Example 39

### 9-Deoxy-9β-chloro-6-thia-3, 4, 5, 16, 17, 18, 19, 20-octanol-2, 6-inter-m-phenylene-15-cyclohexyl-PGF₁α ethyl ester (Compound 55)

(1) To a toluene (24 ml) solution of (3R, 4R)-2-methylene-3-[(3S)-3-(tert-butyldimethylslloxy) -3-cyclohexylprop-1-ynyl]-4-(tert-butyldimethylsilox y)cyclopentane-1-one (4.64 g) obtained in Example 1(1) was added 3-mercaptophenylacetic acid ethyl ester (1.32 g), followed by stirring at room temperature for a week. The reaction solution was concentrated under reduced pressure, and the resulting residue was purified by silica column chromatography (developing solvent: hexane: ethyl acetate = 25: 1) to give 6-thia-3, 4, 5, 16, 17, 18, 19, 20-octanol-2, 6-inter-m-phenylene-15-cyclohexyl-PGE₁ ethyl ester 11, 15-bis(tert-butyldimethylsilyl ether) (980 mg).
   ¹H-NMR(CDCl₃, 300MHz) δppm; 0.05 (s, 3H), 0.06 (s, 3H), 0.09 (s, 3H), 0.12 (s, 3H), 0.72-1.90 (m, 11H), 0.87 (s, 9H) 0.88(s, 9H), 1.12 (t, J=7.2Hz, 3H), 2.23 (dd, J=18.1, 4.9Hz, 1H), 2.41-2.55 (m, 1H), 2.72 (dd, J=18.1, 6.1Hz, 1H), 3.07-3.39 (m, 1H), 3.14 (dd, J=13.3, 8.6Hz, 1H), 3.33 (dd, J=13.3, 4.7Hz, 1H), 3.57 (s, 2H), 4.05 (dd, J=6.4, 1.7Hz, 1H), 4.15 (q, J=7.2Hz, 2H), 4.35-4.42 (m, 1H), 7.06-7.32 (m, 4H)
   IR (neat) 2929, 2856, 2236, 1746, 1594, 1575, 1472, 1464, 1451, 1389, 1336, 1253, 1101, 1065, 1033, 1006, 940, 898, 837, 778, 689 cm⁻¹
(2) Following the substantially same manner as in Example 1(3) using the compound obtained in (1) above, thereby 6-thia-3, 4, 5, 16, 17, 18, 19, 20-octanol-2, 6-inter-m-phenylene-15-cyclohexyl-PGF₁α ethyl ester 11, 15-bis(tert-butyldimethylsilyl ether) was obtained.
   ¹H-NMR(CDCl₃, 300MHz) δ ppm; 0.07 (s, 3H), 0.09 (2s, 6H), 0.10, (s, 3H), 0.73-2.15 (m, 14H), 0.88 (s, 9H), 0.89 (s, 9H), 1.26 (t, J=7.1Hz, 3H), 2.46-2.67 (m, 2H), 3.14-3.31 (m, 1H), 3.57 (s, 2H), 4.04-4.29 (m, 3H), 4.15 (q, J=7.1Hz, 2H), 7.02-7.30 (m, 4H)
   IR(neat) : 3514, 2929, 2855, 2232, 1740, 1593, 1574, 1472, 1451, 1389, 1369, 1336, 1252, 1100, 1062, 1006, 965, 926, 898, 837, 778, 688 cm⁻¹
(3) Following the substantially same manner as in Example 1(4) using the compound obtained in (2) above, thereby 9-deoxy-9β-chloro-6-thia-3, 4, 5, 16, 17, 18, 19, 20-octanol-2, 6-inter-m-phenylene-15-cyclohexyl-PGF₁α ethyl ester 11, 15-bis(tert-butyldimethylsilyl ether) was obtained.
   ¹H-NMR(CDCl₃, 300MHz) δ ppm: 0.06 (s, 3H), 0.07 (s, 3H), 0.08 (s, 3H), 0.09 (s, 3H), 0.81-1.89 (m, 11H), 0.88, (s, 9H), 0.89 (s, 9H), 1.25 (t, J=7.1Hz, 3H), 2.12-2.22 (m, 2H), 2.38-2.44 (m, 1H), 2.62 (ddd, J=8.9, 4.7, 1.7Hz, 1H), 3.16-3.30 (m, 2H, 3.57 (s, 2H), 4.07 (dd, J=6.2, 1.7Hz, 1H), 4.10-4.31 (m, 2H), 4.15 (q, J=7.1Hz, 2H), 7.08-7.32 (m, 4H)
   IR (neat) : 2929, 2856, 2235, 1740, 1594, 1575, 1472, 1450, 1389, 1362, 1337, 1252, 1155, 1100, 1034, 1006, 927, 898, 837, 778, 688 cm⁻¹
(4) Following the substantially same manner as in Example 1(5) using the compound obtained in (3) above, thereby the title compound was obtained.
   ¹H-NMR (CDCl₃, 300MHz) δ ppm; 0.89-252 (m, 16H), 1.26 (t,J=7.2Hz, 3H), 2.61 (ddd, J=9.6, 6.1, 1.9Hz, 1H), 3.17-3.32 (m, 2H), 3.59(s, 2H), 4.08-4.27 (m, 2H), 4.16 (q, J=7.2Hz, 2H), 4.32-4.41 (m, 1H), 7.08-7.32 (m, 4H)
   IR (neat) : 3400, 2980, 2928, 2853, 2236, 1733, 1593, 1574, 1476, 1450, 1428, 1369, 1256, 1154, 1084, 1030, 894, 833, 761, 690, 578 cm⁻¹

### Example 40

### 9-Deoxy-9β-chloro-6-thia-3, 4, 5, 16, 17, 18, 19, 20-octanol-2, 6-inter-m-phenylene-15-cyclohexyl-PGF₁α (Compound 56)

Following the substantially same manner as in Example 2 using the compound obtained in Example 39(4), thereby the title compound was obtained.
¹H-NMR(CDCl₃, 300MHz) δ ppm ; 0.89-1.87 (m, 11H), 2.14-2.48 (m, 3H), 2.55 (ddd, J=9.9, 9, 6.5, 1.9Hz, 1H), 3.13 (dd, J=14.1, 5.4Hz, 1H), 3.28 (dd, J=14.1, 5.1Hz, 1H), 3.61 (s, 2H), 3.94-4.67 (m, 5H), 4.11 (dd, J=6.1, 1.9Hz, 1H), 7.05-7.42 (m, 4H)
IR (neat) : 3368, 2928, 2853, 2237, 1712, 1593, 1574, 1476, 1450, 1261, 1154, 1083, 894, 832, 758, 689, 613 cm⁻¹

### Example 41

### 9-Deoxy-9β-chloro-6-thia-3, 4, 16, 17, 18, 19, 20-heptanol-2,5-inter-m-phenylene-15-cyclohexyl-PGF₁α ethyl ester (Compound 81)

(1) In Example 39(1), following the substantially same manner as in Example 39(1) using 3-mercaptomethylphenylacetic acid ethyl ester in place of 3-mercaptophenylacetic acid ethyl ester, thereby 6-thia-3, 4, 16, 17, 18, 19, 20-heptanol-2, 5-inter-m-phenylene-15-cyclohexyl-PGE₁ ethyl ester 11, 15-bis(tert-butyldimethylsilyl ether) was obtained.
   ¹H-NMR (CDCl₃, 300MHz) δppm; 0.07 (s, 3H), 0.08 (s, 3H), 0.09 (s, 3H), 0.12 (s, 3H), 0.73-1.93 (m, 11H), 0.88 (s, 9H), 0.89 (s, 9H), 1.25 (t, J=7.2Hz, 3H), 2.21 (dd, J=17.9, 6.1Hz, 1H), 2.43-2.53 (m, 1H), 2.66-2.87 (m, 3H), 3.12-3.19 (m, 1H), 3.60 (s, 2H), 3.68 (d, J=13.5Hz, 1H), 3.74 (d, J=13.5Hz, 1H), 4.08 (dd, J=5.0, 1.6Hz, 1H), 4.15 (q, J=7.2Hz, 2H), 4.29-4.39 (m, 1H), 7.13-7.34 (m, 4H)
   IR(neat) : 2929, 856, 2236, 1747, 1608, 1472, 1464, 1449, 1369, 1337, 1256, 1149, 1110, 1065, 1033, 1006, 940, 898, 838, 778, 712, 670, 588 cm⁻¹
(2) Following the substantially same manner as in Example 1(3) using the compound obtained in (1) above, thereby 6-thia-3, 4, 16, 17, 18, 19, 20-heptanol-2,5-inter-m-phenylene-15-cyclohexyl-PGF₁α ethyl ester 11, 15-bis(tert-butyldimethylsilyl ether) was obtained.
   ¹H-NMR (CDCl₃, 300MHz) δ ppm; 0.07 (s, 3H), 0.08 (s, 3H), 0.09 (s, 3H), 0.10 (s, 3H) 0.78-2.15 (m, 14H), 0.87 (s, 9H), 0.90 (s, 9H), 1.26 (t, J=7.2Hz, 3H), 2.44-2.59 (m, 2H), 2.67-2.79 (m, 3H), 3.60 (s, 2H), 3.72 (d, J=13.5Hz, 1H) 3.77 (d, J=13.5Hz, 1H), 4.03-4.26 (m, 2H), 4.07 (dd, J=6.5, 1.8Hz, 1H), 4.15 (q, 1=7.2Hz, 2H), 7.10-7.25 (m, 4H)
   IR(neat) : 3468, 2929, 2855, 2232, 1739, 1608, 1472, 1449, 1387, 1337, 1256, 1110, 1062, 927, 898, 838, 778, 710, 669 cm⁻¹
(3) Following the substantially same manner as in Example 1(4) using the compound obtained in (2) above, thereby 9-deoxy-9β-chloro-6-thia-3, 4, 16, 17, 18, 19, 20-heptanol-2, 5-inter-m-phenylene-15-cyclohexyl-PGF₁α ethyl ester 11, 15-bis(tert-butyldimethylsilyl ether) was obtained.
   ¹H-NMR (CDCl₃, 300MHz) δ ppm; 0.06 (s, 3H), 0.07 (s, 3H), 0.08 (s 3H), 0.11 (s, 3H), 0.80-1.90 (m, 11H), 0.87 (s, 9H) 0.90 (s, 9H), 1.25 (t, J=7.2Hz, 3H) 2.13-2.39 (m, 3H), 2.53-2.61 (m, 3H), 2.68 (dd, J=13.4, 3.7Hz, 1H), 2.75 (dd, J=13.4, 3.7Hz, 1H), 3.60 (s, 2H), 3.74 (s, 2H), 4.05-4.21 (m, 1H), 4.08 (dd, J=6.1, 1.7Hz, 1H), 4.15(q, J=7.2Hz, 2H), 4.23-4.31 (m, 1H), 7.13-7.32 (m, 4H)
   IR(neat) : 2929, 2855, 2235, 1740, 1608, 1472, 1447, 1389, 1362, 1338, 1256, 1154, 1100, 1034, 1006, 962, 898, 838, 778, 710, 670 cm⁻¹
(4) Following the substantially same manner as in Example 1(5) using the compound obtained in (3) above, thereby the title compound was obtained.
   ¹H-NMR(CDCl₃, 300MHz) δ ppm; 0.94-2.42 (m, 16H), 1.26 (t, J=7.2Hz, 3H), 2.53 (ddd, J=9.7, 6.2, 1.8Hz, 1H), 2.68-2.79 (m, 2H), 3.61 (s, 2H), 3.74 (s, 2H), 4.07-4.22 (m, 1H), 4.16 (q, J=7.2Hz, 2H), 4.31-4.41 (m, 1H), 7.13-7.32 (m, 4H)
   IR (neat) : 3400, 2980, 2929, 2853, 2236, 1734, 1608, 1590, 1489, 1447, 1370, 1262, 1154, 1085, 1030, 894, 758, 711, 579 cm⁻¹

### Example 42

### 9-Deoxy-9β-chloro-6-thia-3, 4, 16, 17, 18, 19, 20-heptanol-2, 5-inter-m-phenylene-15-cyclohexyl-PGF₁α (Compound 82)

Following the substantially same manner as in Example 2 using the compound obtained in Example 41(4), thereby the title compound was obtained.
¹H-NMR (CDCl₃, 300MHz) δ ppm; 0.94-1.88 (m, 14H), 2.08-2.38 (m, 3H), 2.45 (ddd, J=9.6, 6.5, 1.9Hz, 1H), 2.63(dd, J=13.3, 5.5Hz, 1H), 2.74(dd, J=13.3, 5.5Hz, 1H), 3.63 (s, 2H), 3.74 (s, 2H), 4.03-4.17 (m, 2H), 4.28-4.37 (m, 1H), 7.08-7.36 (m, 4H)
IR(neat) : 3368, 2928, 2853, 2236, 1712, 1608, 1590, 1489, 1449, 1413, 1262, 1154, 1084, 1007, 958, 894, 758, 708, 668, 614 cm⁻¹

### Example 43

### 9-Deoxy-9β-chloro-3, 4, 5, 16, 17, 18, 19, 20-octanol-2, 6-inter-m-phenylene-15-cyclohexyl-PGF₁α ethyl ester (Compound 13)

(1) (1E, 3S)-1-Iodo-3-(tert-butyldimethylsiloxy)-3-cyclohexyl -1-propene (2.66 g) was dissolved in 28 ml of ether, tert-butyllithium (1.7 M, pentane solution, 8.24 ml) was added at -78 degree C followed by stirring at the same temperature for 1 hour, lithium 2-thienylcyanocuprate (0.25 M, tetrahydrofuran solution, 39.2 ml) was added followed by further stirring at the same temperature for 20 minutes, and
   (4R)-2-(N,N-diethylamino)methyl-4-(tert-butyldimethy lsiloxy)cyclopent-2-en-1-one (0.25 M, ether solution, 28 ml) was added followed by elevating temperature to 0 degree C in 1.5 hours under stirring. To the reaction solution was added hexane (70 ml) and a saturated aqueous ammonium chloride solution (105 ml), and extracted with hexane and washed with a saturated aqueous sodium chloride solution, dried, concentrated and purified by silica gel column chromatography (developing solvent; hexane: ethyl ester of acetate = 30: 1) to give (3R, 4R)-2-methylene-3-[(1E, 3S)-3-(tert-butyldimethylsiloxy)-3-cyclohexyl-1-prop enyl]-4-(tert-butyldimethylsiloxy)cyclopentane-1-one (910 mg).
   ¹H-NMR(CDCl₃, 300MHz) δ ppm; 0.00 (s, 3H), 0.01 (s, 3H), 0.04 (s, 3H), 0.07 (s, 3H), 0.73-1.89 (m, 11H), 0.88 (s, 9H), 0.90 (s, 9H), 2.33 (dd, J=17.9, 6.3Hz, 1H), 2.65 (dd, J=17.9, 6.3Hz, 1H), 3.27-3.91 (m, 2H), 4.07-4.20 (m, 1H), 5.25 (dd, J=2.5, 1.0Hz, 1H), 5.47 (ddd, J=15.9, 7.2, 0.8Hz, 1H), 5.61 (dd, J=15.5, 5.1Hz, 1H), 6.12 (dd, J=2.9, 1.0Hz, 1H)
   IR(neat): 2954, 2929, 2856, 1734, 1642, 1472, 1451, 1388, 1361, 1253, 1113, 1071, 1006, 973, 943, 923, 900, 837, 776, 690 cm⁻¹
(2) Following the substantially same manner as in Example 1(2) using the compound obtained in (1) above in place of (3R, 4R)-2-methylene-3-[(3S)-3-(tert-butyldimethylsiloxy) -3-cyclohexxlprop-1-ynyl]-4-(tert-butyldimethylsilox y)cyclopentane-1-one, thereby 3, 4, 5, 16, 17, 18, 19, 20-octanol-2, 6-inter-m-phenylene-15-cyclohexyl-PGE₁ ethyl ester 11, 15-bis(tert-butyldimethylsilyl ether) was obtained.
   ¹H-NMR(CDCl₃, 300MHz) δppm; -0.01 (s, 3H), 0.04 (s, 3H), 0.06 (s, 3H), 0.07 (s, 3H), 0.78-2.06 (m, 14H), 0.88 (s, 9H), 0.90(s. 9H), 1.25(t, J=7.1Hz, 3H), 2.21 (dd, J=18.3, 7.8Hz, 1H), 2.45-2.87 (m, 4H), 3.57(s, 2H), 3.81-3.89 (m, 1H), 4.04-4.19(m, 1H), 4.14(q, J=7.1Hz, 2H), 5.49 (dd, J=15.4, 6.8Hz, 1H), 5.57(dd, J=15.4, 4.9Hz, 1H), 7.03-7.28 (m, 4H)
   IR (neat): 2929, 2856, 1744, 1609, 1472, 1463, 1450, 1368, 1252, 1152, 1098, 1034, 1006, 973, 940, 881, 838, 776, 700, 670 cm⁻¹
(3) Following the substantially same manner as in Example 1(3) using the compound obtained in (2) above, thereby 3, 4, 5, 16, 17, 18, 19, 20-octanol-2, 6-inter-m-phenylene-15-cyclohexyl-PGF₁α ethyl ester 11, 15-bis(tert-butyldimethylsilyl ether) was obtained.
   ¹H-NMR(CDCl₃, 300MHz) δ ppm; -0.02 (s, 3H), 0.02 (s, 3H), 0.06 (s, 6H), 0.74-2.06 (m, 16H), 0.88 (s, 9H), 0.89 (s, 9H), 1.25 (t, J=7.1Hz, 3H), 1.56 (d, J=3.9Hz, 1H), 2.20-2.33 (m, 2H), 2.49-2.62 (m, 1H), 2.70-2.84 (m, 2H), 3.57 (s, 2H), 3.70-3.81 (m, 1H), 4.02-4.22 (m, 2H), 4.14 (q, J=7.1Hz, 2H), 5.30 (dd, J=15.5, 8.4Hz, 1H), 5.41 (dd, J=15.5, 6.3Hz, 1H), 7.06-7.27 (m, 4H)
   IR(neat) : 3523, 2929, 2855, 1740, 1608, 1472, 1463, 1450, 1388, 1368, 1255, 1100, 1060, 1032, 1005, 973, 837, 775, 701, 668 cm⁻¹
(4) Following the substantially same manner as in Example 1(4) using the compound obtained in (3) above, thereby 9-deoxy-9β-chloro-3, 4, 5, 16, 17, 18, 19, 20-octanol-2, 6-inter-m-phenylene-15-cyclohexyl-PGF₁α ethyl ester 11, 15-bis(tert-butyldimethylsilyl ether) was obtained.
   ¹H-NMR(CDCl₃, 300MHz) δ ppm; -0.01 (s, 3H), 0.03 (s, 3H), 0.04 (s, 3H), 0.06 (s, 3H), 0.79-1.96 (m, 14H), 0.87 (s, 9H), 0.90 (s, 9H), 1.25 (t, J=7.1Hz, 3H), 2.05-2.31 (m, 3H), 2.54-2.86 (m, 2H), 3.55 (s, 2H), 3.75-3.85 (m, 1H), 4.02-4.20 (m, 2H), 4.15 (q, J=7.1Hz, 2H), 5.37-5.52 (m, 2H), 7.03-7.27 (m, 4H)
   IR(neat) : 2929, 2856, 1740, 1609, 1472, 1463, 1450, 1388, 1362, 1255, 1097, 1034, 1006, 974, 940, 899, 837, 776, 700, 670 cm⁻¹
(5) Following the substantially same manner as in Example 1(5) using the compound obtained in (4) above, thereby the title compound was obtained.
   ¹H-NMR(CDCl₃, 300MHz) δppm; 0.84-2.36 (m, 19H), 1.26 (t, J=7.2Hz, 3H), 2.60-2.84 (m, 2H), 3.58 (s, 2H), 3.77-3.87 (m, 1H), 4.04-4.19 (m, 2H), 4.15 (q, J=7.2Hz, 2H), 5.50 (dd, J=15.1, 7.6Hz, 1H), 5.59 (dd, J=15.1, 6.3Hz, 1H), 7.03-7.28 (m, 4H)
   IR(neat) : 3368, 1925, 2853, 1735, 1608, 1590, 1449, 1401, 1369, 1152, 1084, 1032, 972, 892, 768, 701 cm⁻¹

### Example 44

### 9-Deoxy-9β-chloro-3, 4, 5, 16, 17, 18, 19, 20-octanol-2, 6-inter-m-phenylene-15-cyclohexyl-PGF₁α (Compound 15)

Following the substantially same manner as in Example 2 using the compound obtained in Example 43(5), thereby the title compound was obtained.
¹H-NMR(CDCl₃, 300MHz) δppm; 0.84-2.98 (m, 22H), 3.61 (s, 2H), 3.75-3.94 (m, 1H), 4.02-4.18 (m, 2H), 5.28-5.61 (m, 2H), 7.04-7.30 (m, 4H)
IR(neat) : 3368, 2925, 2853, 1712, 1609, 1489, 1449, 1402, 1260, 1153, 1084, 1000, 974, 892, 758, 703, 616 cm⁻¹

### Example 45

### 9-Deoxy-9β-chloro-3, 4, 5, 16, 17, 18, 19, 20-octanol-2, 6-inter-o-phenylene-15-cyclohexyl-PGF₁α ethyl ester (Compound 18)

(1) In Example 1(2), following the substantially same manner as in Example 1(2) using 2-carboethoxymethylbenzylzinc(II) bromide in place of 3-carboethoxymethylbenzylzinc(II) bromide, and using the compound obtained in Example 43(1) in place of (3R, 4R)-2-methylene-3-[(3S)-3-(tert-butyldimethylsiloxy) -3-cyclohexylprop-1-ynyl]-4-(tert-butyldimethylsilox y)cyclopentane-1-one, thereby 3, 4, 5, 16, 17, 18, 19, 20-octanol-2, 6-inter-o-phenylene-15-cyclohexyl-PGE₁ ethyl ester 11, 15-bis(tert-butyldimethylsilyl ether) was obtained.
   ¹H NMR(CDCl₃, 300MHz) δppm; -0.01 (s, 3H), 0.04 (s, 3H), 0.06 (s, 3H), 0.07 (s, 3H), 0.67-2.09, (m, 14H), 0.88 (s, 9H), 0.90 (s, 9H), 1.25 (t, J=7.1Hz, 3H), 2.21 (dd, J=18.4, 8.2Hz, 1H), 2.48-2.76 (m, 3H), 2.83 (ddd, J=13.7, 11.3, 5.4Hz, 1H). 3.68 (s, 2H), 3.82.-3.92 (m, 1H), 4.06-4.21 (m, 1H), 4.15 (q, J=7.1Hz, 2H), 5.51 (dd, j=15.7, 7.3Hz, 1H), 5.60 (dd, J=15.7, 5.0Hz, 1H), 7.09-7.30 (m, 4H)
   IR(neat): 2929, 2856, 1741, 1472, 1451, 1368, 1334, 1252, 1155, 1100, 1032, 1006, 973, 940, 923, 883, 838, 776, 670 cm⁻¹
(2) Following the substantially same manner as in Example 1(3) using the compound obtained in (1) above, thereby 3, 4, 5, 16, 17, 18, 19, 20-octanol-2, 6-inter-o-phenylene-15-cyclohexyl-PGF₁α ethyl ester 11, 15-bis(tert-butyldimethylsilyl ether) was obtained.
   ¹H-NMR(CDCl₃, 300MHz) δppm; -0.01 (s, 3H), 0.03 (s, 3H),0.05 (s, 3H), 0.06 (s, 3H), 0.80-1.98 (m, 16H), 0.87 (s, 9H), 0.89 (s, 9H), 1.24 (t, J=7.1Hz, 3H), 2.21-2.32 (m, 1H), 2.46-2.61 (m, 1H), 2.78-2.91 (m, 2H), 3.65 (d, J=15.3Hz, 1H), 3.70 (d, J=15.3Hz, 1H), 3.73-3.80 (m, 1H), 4.02-4.08 (m, 1H), 4.14 (q, J=7.2Hz, 2H), 4.18-4.27 (m, 1H), 5.32 (dd, J=15.4, 8.5Hz, 1H), 5.42 (dd, J=15.4, 5.9Hz, 1H), 7.09-7.24 (m, 4H)
   IR(neat): 3436, 3118, 2929, 2855, 1736, 1472, 1451, 1386, 1255, 1155, 1085, 973, 899, 837, 776 cm⁻¹
(3) Following the substantially same manner as in Example 1(4) using the compound obtained in (2) above, thereby 9-deoxy-9β-chloro-3, 4, 5, 16, 17, 18, 19, 20-octanol-2, 6-inter-o-phenylene-15-cyclohexyl-PGF₁α ethyl ester 11, 15-bis(tert-butyldimethylsilyl ether) was obtained.
   ¹H-NMR (CDCl₃, 300MHz) δppm; -0.01 (s, 3H), 0.03 (s, 3H), 0.04 (s, 3H), 0.05 (s, 3H), 0.78-1.98 (m, 14H), 0.87 (s, 9H), 0.89 (s, 9H), 1.25 (t, J=7.2Hz, 3H), 2.06-2.32 (m, 3H), 2.57-2.91 (m, 2H), 3.63 (d,J=15.2Hz, 1H), 3.65 (d, J=15.2Hz, 1H), 3.76-3.85 (m, 1H), 4.04-4.21 (m, 1H), 4.16 (q, J=7.1Hz, 2H), 5.22-5.56 (m, 2H), 7.10-7.28 (m, 4H)
   IR (neat) : 3118, 2929, 2856, 1740, 1472, 1463, 1451, 1386, 1253, 1155, 1097, 1032, 1006, 974, 899, 837, 776, 670 cm⁻¹
(4) Following the substantially same manner as in Example 1(5) using the compound obtained in (3) above, thereby the title compound was obtained.
   ¹H-NMR(CDCl₃, 300MHz) δ ppm; 0.82-2.38 (m, 19H), 1.25 (t, J=7.1Hz, 3H), 2.61-2.90 (m, 2H), 3.63 (s, 2H), 3.78-3.88 (m, 1H), 4.08-4.21 (m, 2H), 4.15 (q, J=7.1Hz, 2H) 5.40-5.67 (m, 2H), 7.12-7.27 (m, 4H)
   IR(neat) : 3368, 2925, 2852, 1734, 1493, 1450, 1368, 1256, 1157, 1095, 1030, 972, 892, 755 cm⁻¹

### Example 46

### 9-Deoxy-9β-chloro-3, 4, 5, 16, 17, 18, 19, 20-octanol-2, 6-inter-o-phenylene-15-cyclohexyl-PGF₁α (Compound 19)

Following the substantially same manner as in Example 2 using the compound obtained in Example 45(4), thereby the title compound was obtained.
¹H-NMR (CDCl₃, 300MHz) δppm; 0.78=2.89 (m, 22H), 3.59 (d, J=16.1Hz, 1H), 3.71 (d, J=16.1Hz, 1H), 3. 85-3. 97 (m, 1H), 4.05-4.34 (m, 2H), 5.36-5.68 (m, 2H), 7.13-7.27 (m, 4H)
IR(neat): 3400, 2929, 1714, 1494, 1450, 1385, 1262, 1073, 757, 616 cm⁻¹

### Example 47

### (13Z)-9-Deoxy-9β-chloro-3, 4, 5, 16, 17, 18, 19, 20-octanol-2, 6-inter-o-phenylene-15-cyclohexyl-PGF₁α ethyl ester (Compound 93)

An ethanol solution (4 ml) of the compound obtained in Example 19 (100 mg) and 10% palladium-activated carbon (7 mg) was stirred at room temperature for 15 minutes under an atmosphere of hydrogen. The reaction solution was filtered, concentrated, and the resulting crude product was purified by silica gel column chromatography (developing solvent; hexane: ethyl acetate = 3:1) to give the title compound (59 mg).
¹H-NMR(CDCl₃, 300MHz) δppm; 0.81-2.03 (m, 15H), 1.25 (t, J=7.2Hz, 3H), 2.18-2.37 (m, 2H), 2.50-2.82 (m, 4H) 3.64 (s, 2H), 4.05-4.20 (m, 3H), 4.14 (q, J=7.2Hz, 2H), 5.36-5.47 (m, 1H), 5.61 (dd, J=11.0, 8.9Hz, 1H), 7.12-7.28 (m, 3H)

### Example 48

### 9-Deoxy-9β-chloro-3, 4, 5, 16, 17, 18, 19, 20-octanol-2, 6-inter-o-phenylene-15-cyclohexyl-13, 14-dihydro-PGF₁α (Compound 3)

(1) In Example 43(1), following the substantially same manner as in Example 43(1) using (3R)-1-iodo-3-(tert-butyldimethylsiloxy)-3-cyclohexy Ipropane in place of (1E, 3S)-1-iodo-3-(tert-butyldimethylsiloxy)-3-cyclohexyl propene, thereby (3R, 4R)-2-methylene-3-[(3R)-3-(tert-butyldimethylsiloxy) -3-cyclohexyl-1-propyl]-4-(tert-butyldimethylsiloxy) cyclopentane-1-one was obtained.
   ¹H-NMR(CDCl₃, 300MHz) δ ppm; 0.02 (s, 3H), 0.03 (s, 3H), 0.06 (s, 3H), 0.08 (s, 3H), 0.55-1.85 (m, 15H), 0.88 (s, 9H), 0.89 (s, 9H), 2.24-2.38 (m, 1H), 2.53-2.72 (m, 1H), 2.62 (dd, J=18.1, 5.9Hz, 1H), 3.37-3.49 (m, 1H), 4.06-4.17 (m, 1H), 5.27-5.32 (m, 1H), 6.08 (d, J=2.2Hz, 1H)
   IR(neat) : 2954, 2929, 2856, 1734, 1642, 1473, 1463, 1362, 1256, 1089, 1072, 1006, 939, 836, 775, 670 cm⁻¹
(2) In Example 1(2), following the substantially same manner as in Example 1(2) using 2-carboethoxymethylbenzylzinc(II) bromide in place of 3-carboethoxymethylbenzylzinc(II) bromide and using the compound obtained in (1) above in place of (3R, 4R)-2-methylene-3-[(3S)-3-(tert-butyldimethylsiloxy) -3-cyclohexylprop-1-ynyl]-4-(tert-butyldimethylsilox y)cyclopentane-1-one, thereby 3, 4, 5, 16, 17, 18, 19, 20-octanol-2, 6-inter-o-phenylene-15-cyclohexyl-13, 14-dihydro-PGE₁ ethyl ester 11, 15-bis(tert-butyldimethylsilyl ether) was obtained.
   ¹H-NMR (CDCl₃, 300MHz) δ ppm; 0.02 (s, 3H), 0.03 (s, 3H), 0.06 (s, 3H), 0.09 (s, 3H), 0.78-1.98 (m, 19H), 0.89 (s, 18H), 1.25 (t, J=7.1Hz, 3H), 2.19 (dd, J=18.1, 6.0Hz, 1H), 2.53-2.85 (m, 2H), 2.60 (dd, J=18.1, 6.3Hz, 1H), 3.34-3.44 (m, 1H), 3.64 (d, J=15.5Hz, 1H), 3.69 (d, J=15.5Hz, 1H), 4.02-4.20 (m, 1H), 4.15 (q, J=7.1Hz, 2H), 7.11-7.27 (m, 4H)
   IR(neat) : 2929, 2855, 1741, 1472, 1463, 1367, 1332, 1253, 1155, 1104, 10.72, 1034, 1006, 939, 876, 836, 774, 669 cm⁻¹
(3) Following the substantially same manner as in Example 1(3) using the compound obtained in (2) above, thereby 3, 4, 5, 16, 17, 18, 19, 20-octanol-2, 6-inter-o-phenylene-15-cyclohexyl-13, 14-dihydro-PGF₁α ethyl ester 11, 15-bis(tert-butyldimethylsilyl ether) was obtained.
   ¹H-NMR (CDCl₃, 300MHz) δppm; 0.02 (s, 3H), 0.03 (s, 3H), 0.07 (s, 3H), 0.08 (s, 3H), 0.78-2.06 (m, 20H), 0.87 (s, 9H), 0.88 (s, 9H), 1.24 (t, J=7.2Hz, 3H), 2.55 (ddd, J=13.7, 11.0, 5.5Hz, 1H), 2.88 (ddd, J=13.7, 11.3, 4.7Hz, 1H), 3.07 (d, J=10.6Hz, 1H), 3.31-3.40 (m, 1H), 3.66 (d, J=15.3Hz, 1H), 3.75 (d, J=15.3Hz, 1H), 3.95-4.01 (m, 1H), 4.07-4.26 (m, 1H), 4.14 (q, J=7.2Hz, 2H), 7.11-7.27 (m, 4H)
   IR(neat) : 3524, 2929, 2855, 1737, 1498, 1472, 1463, 1451, 1387, 1367, 1331, 1255, 1155, 1088, 1071, 1032, 1006, 872, 836, 774, 666 cm⁻¹
(4) Following the substantially same manner as in Example 1(4) using the compound obtained in (3) above, thereby 9-deoxy-9β-chloro-3, 4, 5, 16, 17, 18, 19, 20-octanol-2, 6-inter-o-phenylene-15-cyclohexyl-13, 14-PGF₁α ethyl ester 11, 15-bis(tert-butyldimethylsilyl ether) was obtained, and further, following the substantially same manner as in Example 1(5), thereby 9-deoxy-9β-chloro-3, 4, 5, 16, 17, 18, 19, 20-octanol-2, 6-inter-o-phenylene-15-cyclohexyl-13, 14-dihydro-PGF₁α ethyl ester was obtained.
   ¹H-NMR (CDCl₃, 300MHz) δppm; 0.90-1.97 (m, 21H), 1.25 (d, J=7.2Hz, 3H), 2.16 (ddd, J=13.7, 7.6, 5.9Hz, 1H), 2.21 (ddd, J=13.7, 6.9, 4.8Hz, 1H), 2.64-2.84 (m, 2H), 3.30-3.48 (m, 1H), 3.66 (s, 2H), 4.07-4.20 (m, 1H), 4.15 (q, J=7.2Hz, 2H), 7.12-7.29 (m, 4H)
   IR(neat) : 3384, 2926, 2854, 1732, 1494, 1450, 1385, 1368, 1335, 1258, 1157, 1096, 1030, 892, 753 cm⁻¹
(5) Following the substantially same manner as in Example 2 using the compound obtained in (4) above, thereby the title compound was obtained.
   ¹H-NMR (CDCl₃, 300MHz) δppm; 0.85-1.90 (m, 21H), 2.17 (dd, J=7.2, 5.8Hz, 1H), 2.65 (ddd, J=13.8, 10.9, 5.5Hz, 1H), 2.76 (ddd, J=13.8, 10.9, 6.2Hz, 1H), 3.40-4.36 (m, 43H), 3.62 (d, J=16.0Hz, 1H), 3.72(d, J=16.0Hz, 1H), 7.13-7.30 (m, 4H)
   IR (neat): 3384, 3062, 3021, 2926, 2854, 2660, 1714, 1494, 1463, 1450, 1416, 1385, 1336, 1295, 1237, 1163, 1088, 1065, 1048, 1020, 969, 893, 842, 796, 755, 668, 618, 560 cm⁻¹

### Test Example 1 [PGD₂ receptor (DP receptor) binding test]

The test was conducted in accordance with the method of TsurI et al. (J. Med. Chem., 40, 3504-3507 (1997)).

### (1) Preparation of human platelet membrane fraction

A sample of blood drawn from a healthy adult using a plastic syringe was placed in a plastic test tube containing 3.8% sodium citrate, and after gently mixing by overturning centrifuged at room temperature at 180 x g for 20 minutes to obtain platelet-rich plasma (PRP) as supernatant. The PRP was further centrifuged at room temperature at 800 x g for 20 minutes to obtain platelet as precipitate. The precipitate obtained was homogenized in a buffer solution (150 mM NaCl, 5 mM KCl, 5 mM glucose, 10 mM Tris/HCl (pH 7.4)), and twice centrifuged at 105,000 x g for 20 minutes at 4 degree C to obtain platelet membrane fraction as precipitate. The membrane fraction was suspended in a preservation solution (250 mM sucrose, 1 mM EGTA, 10 mM Tris/HCl (pH 7.4)) at 3 mg/ml and was kept frozen at -80 degree C until it was used for binding experiments.

### (2) Binding test to PGD₂ receptor

The human platelet membrane fraction (0.1 mg), 5 nM [3H] PGD₂, and each compound was added to 200 µl of binding reaction solution (50 mM Tris/HCl (pH 7.4), 1 mM EDTA, 10 mM MgCl₂), and reaction was conducted at 4 degree C for 90 minutes. After reaction, the mixture was filtered under vacuum using a glass fiber filter paper, and after rinsing several times with cold physiological sodium chloride solution, residual radio activity on the filter paper was measured. The quantity of nonspecific binding in the present experiment was calculated as the quantity of radioactivity when a similar experiment was conducted in the presence of 10 µM PGD₂, and the quantity of specific binding was given as the value of quantity of total binding minus quantity of nonspecific binding. The binding inhibition activity of each compound was calculated as an inhibition constant (Ki value) by drawing a substitutive curve using the quantity of binding (%) in the presence of each compound taking the quantity of specific binding in the absence of the compound as 100%.

The result was shown in the table below.

**Table 2**

| Compound | Inhibition constant Ki (nM) |
|---|---|
| Compound 38 | 0.8 |
| Compound 51 | 2.3 |

The above result showed that the compounds of the present invention were strongly bound to DP receptor.

### Test experiment 2 [Competitive inhibition activity test to PGD₂ receptor]

EBTr[NBL-4] cells derived from embryonic bovine trachea were seeded on a micro-plate with 96 wells, at 8000 cells/well, and used for the experiment after culturing for 2 days in the presence of MEM culture medium (containing 10% calf cerum). The experiment was conducted in a MEM culture medium containing 500 µM IBMX and 10% calf serum. 100 µl of culture medium containing PGD₂ and the test compound was added to initiate the reaction, and after reaction at 37 degree C for 15 minutes, the culture medium was removed and ice-chilled PBS was added to terminate the reaction. Further, after removal of PBS, 200 µl of cytolytic agent (Lysis reagent 1) in cAMP enzymeImmunoassay system (Amersham) was added and cytolysis was brought about by shaking at room temperature for 10 minutes. The quantity of cAMP in the cytolytic agent was determined in accordance with the method described in the CAMP enzymeImmunoassay system. The inhibition activity of the test compound was calculated with the pA₂ value as an indicator using the IC₅₀ value when cAMP production quantity in the presence of 100 nM, 1µM PGD₂ was taken as 100%.

The results were shown in a table.

**Table 3**

| Compound | Competitive inhibition activity IC₅₀ (µM) | |
|---|---|---|
| | PGD₁ (100 nM) | PGD₂ (1 µM) |
| Compound 38 | 0.73 | 1.16 |
| Compound 51 | 3.42 | 5.37 |

It is apparent from the table that the compounds of the present invention were found to have competitive inhibition activity to DP receptor.

### INDUSTRIAL APPLICABILITY

The compound of the present invention has an excellent DP antagonist activity and, therefore, is useful against disease such as allergic rhinitis, nasal obstruction, asthma, allergic conjunctivitis, systemic mastocytosis and disorder of systemic mast cell activation.

## Claims

1. A prostaglandin derivative represented by the formula, 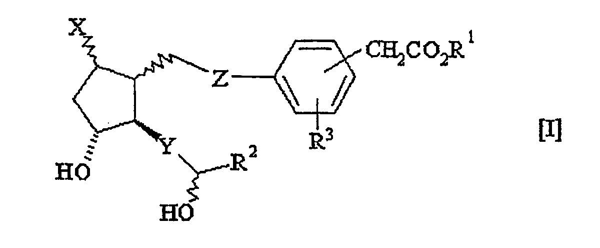 [wherein X is a halogen atom; Y is an ethylene group, a vinylene group, or an ethynylene group; Z is a group represented by -(CH₂)ₘ-, -O(CH₂)ₙ- or -S(O)ₚ-(CH₂)ₙ- (m is an integer of 0 to 3; n is an integer of 0 to 2; and p is an integer of 0 to 2); R¹ is a hydrogen atom, a C₁₋₅ alkyl group, or a substituted C₁₋₅ alkyl group; R² is a C₃₋₁₀ cycloalkyl group, a C₃₋₁₀ cycloalkyl group substituted by a C₁₋₄ alkyl group, or a C₄₋₁₅ cycloalkylalkyl group; and R³ is a hydrogen atom, a halogen atom, a C₁₋₅ alkyl group or a substituted C₁₋₅ alkyl group],
a pharmaceutically acceptable salt thereof or a hydrate thereof.

2. The prostaglandin derivative according to claim 1, wherein in Formula [I], X is a fluorine atom, chlorine atom or a bromine atom and R² is a C₃₋₁₀ cycloalkyl group or a C₄₋₁₅ cycloalkylalkyl group, the pharmaceutically acceptable salt thereof or the hydrate thereof.

3. The prostaglandin derivative according to any of claims 1 or 2, wherein in Formula [I], Y is a vinylene group or an ethynylene group, the pharmaceutically acceptable salt thereof or the hydrate thereof.

4. The prostaglandin derivative according to any of claims 1 or 2, wherein in Formula [I], Y is an ethynylene group, the pharmaceutically acceptable salt thereof or the hydrate thereof.

5. Use of the prostaglandin derivative or the pharmaceutically acceptable salt thereof or the hydrate thereof according to any of claims 1 to 4, for manufacturing a drug for allergic rhinitis, nasal obstruction, asthma, allergic conjunctivitis, systemic mastocytosis or disorder of systemic mast cell activation.

6. A method of treating allergic rhinitis, nasal obstruction, asthma, allergic conjunctivitis, systemic mastocytosis or disorder of systemic mast cell activation, by administering to the prostaglandin derivative or the pharmaceutically acceptable salt thereof or the hydrate thereof according to any of claims 1 to 4.
